# EUROPEAN PATENT APPLICATION

(11) **EP 3 663 413 A1**
(43) Date of publication of application: **10.06.2020**
(21) Application number: 18210684.9
(22) Date of filing: 06.12.2018
(51) Int. Cl.: C12Q 1/6883

(54) **BIOMARKER FOR DETECTING ZEARALENONE EFFECT**

(71) Applicant: Erber Aktiengesellschaft, 3131 Getzersdorf bei Traismauer (AT)
(72) Inventor: Grenier, Bertrand, 3071 Böheimkirchen (AT); Nagl, Veronika, 3071 Böheimkirchen (AT); Schatzmayr, Gerd, 3430 Tulln (AT); Binder, Eva Maria, 3430 Tulln (AT)
(74) Representative: Schiweck Weinzierl Koch Patentanwälte Partnerschaft mbB

(57) **Abstract**

The present invention relates to a novel method for detecting zearalenone (ZEN) effect comprising determining the expression level of at least one miRNA in a test sample, as well as to a novel use of at least one miRNA for detecting zearalenone (ZEN) effect in a test sample.

## Description

### FIELD OF THE INVENTION

The present invention relates to a novel method for detecting zearalenone (ZEN) effect comprising determining the expression level of at least one miRNA in a test sample, as well as to a novel use of at least one miRNA for detecting zearalenone (ZEN) effect in a test sample.

### DESCRIPTION

Mycotoxins are secondary metabolites of molds, which lead to concentration-dependent adverse health effects in animals. In Northern and Central Europe, mycotoxins produced by field fungi such as *Fusarium graminearum* or *F. culmorum* are most frequently detected in animal feeds (Streit et al. 2012. Toxins, 4, 788-809). Zearalenone (ZEN) is regarded as one of the most relevant *Fusarium* mycotoxins, particularly in pig production. ZEN is a frequent contaminant of maize but can also occur in various other commodities such as wheat, barley or oats. In a recent study, 88% of tested feed samples tested positive for ZEN with median and maximum concentrations of 20 µg/kg and 11,192 µg/kg, respectively (Kovalsky et al. 2016. Toxins, 8, E363). Pigs are especially susceptible to the effects of ZEN, which is attributed to the species-specific metabolism of this mycotoxin (Fink-Gremmels and Malekinejad. 2007. Animal Feed Science and Technology, 137, 326-341). ZEN exhibits low acute toxicity but acts as full and partial agonist on estrogen receptors α and β, respectively (Zinedine et al. 2007. Food and Chemical Toxicology, 45, 1-18). As a consequence, ZEN exposure of pigs results in reproduction and fertility disorders. For example, mammary gland development is triggered in prepuberal gilts fed diets containing 1-5 ppm ZEN. Also, vulvovaginitis, edema of vulva and rectal prolapses are observed (Osweiler, G. 1999. Mycotoxins. In: STRAW, B., DÁLLAIRE, S., MENGELING, W. & TAYLOR, D. (eds.) 8th Diseases of swine. Ames, Iowa, USA: Iowa State University Press; Osweiler, G. D. 2000. Mycotoxins: Contemporary issues of food animal health and productivity. Veterinary Clinics of North America: Food Animal Practice, 16, 511-530). In mature sows, the effects of ZEN are dependent on the concentration in the feed, duration of exposure and phase of gestation, and range from anestrus to a decrease in live embryos, an increase in dead-born piglets and abortions (Kordic et al. 1992. Journal of environmental pathology, toxicology and oncology: official organ of the International Society for Environmental Toxicology and Cancer, 11, 53-55). In newborn piglets of exposed sows, enlargement of external genitalia and a higher incidence of piglets with splay leg and trembling were recorded (Osweiler, G. 1999. Mycotoxins. In: STRAW, B., DÁLLAIRE, S., MENGELING, W. & TAYLOR, D. (eds.) 8th Diseases of swine. Ames, Iowa, USA: Iowa State University Press). In addition, ZEN activates different receptors, most prominently the pregnane X receptor, involved in the regulation of cytochrome P450 isoforms in vitro, thus potentially affecting the phase I metabolism of various endo- and xenobiotics (Ayed-Boussema et al. 2011. Environmental Toxicology and Pharmacology, 31, 79-87).

So far, detection of ZEN mycotoxicosis is mainly based on the observation of clinical signs and feed analysis. Yet, ZEN, as many other mycotoxins, exhibits adverse health effects in livestock before clinical symptoms become evident. Analysis of feed samples is accompanied by certain challenges: Representative feed samples need to be collected taking into account an inhomogeneous distribution of mycotoxins within a lot as well as time point of sampling as feed lots might have changed between sampling and the initial onset of mycotoxin-induced effects. Also, mycotoxin co-contamination of feed samples needs be considered, which can result in additive, synergistic or antagonistic effects in animals (Grenier and Oswald. 2011. World Mycotoxin Journal, 4, 285-313). Moreover, the presence of modified forms of ZEN are not routinely monitored but add up to the total ZEN burden on an individuum (Binder et al. 2017. Toxins, 9, 56). In contrast to feed analyses, the detection of specific biomarkers in biological matrices of swine, such as urine, feces, bile or tissue would allow detection of mycotoxicosis on an individual level. Two types of biomarkers are used in mycotoxin research: Exposure-based biomarkers and mechanism-based biomarkers (Baldwin et al. 2011. World Mycotoxin Journal, 4, 257-270). While the former relate to the measurement of a mycotoxin itself and/or its metabolites, the latter relate to a specific biological response, e.g. increase/decrease in protein levels or cellular metabolite levels, that can be linked to mycotoxin intake. Concerning ZEN, efforts have been taken during the last years to establish an exposure-based biomarker in pigs. Under controlled conditions, numerous studies have investigated effects of increasing toxin concentrations on levels of ZEN and/or its metabolites in blood, urine and bile, but also in feces, liquor, liver or back fat. Usually, highest concentrations of ZEN and its metabolites were recovered from bile and urine, with better correlation coefficients, i.e. ingested ZEN versus recovered ZEN, for bile and urine as matrices than for other matrices such as blood (Doll et al. 2003. Archives of Animal Nutrition, 57, 311-334; Goyarts et al. 2007. Food Additives and Contaminants, 24, 369-380; Brezina et al. 2014. Archives of animal nutrition, 68, 425-447). In accordance, a recent field study failed to correlate ZEN levels in sow feed to those in plasma, as toxin concentrations in plasma were under the limit of quantification in the majority of samples (Van Limbergen et al. 2017. Veterinary Record, 181, 539). Exposure-based biomarkers can still not be recommended for a prediction of ZEN intake under field conditions, since comparable concentrations of ZEN in bile, blood or urine can derive from a broad range of ZEN exposure levels (Dänicke and Winkler. 2015. Food and Chemical Toxicology, 84, 225-249). Most of all, ZEN residues do not provide indications on the severity of adverse health effects induced by ZEN, as demonstrated e.g. by the absence of a correlation between the total ZEN levels in bile and the uterus weight after ZEN exposure (Dänicke and Winkler. 2015. Food and Chemical Toxicology, 84, 225-249). Regarding mechanism-based ZEN biomarkers, several biological parameters, such as hematological and biochemical parameters (Goyarts et al. 2007. Food Additives and Contaminants, 24, 369-380; Gaj 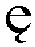 cka et al. 2016. Research in Veterinary Science, 109, 169-180) or mRNA expression (Dai et al. 2016. Animal Reproduction Science, 168, 126-137; Reddy et al. 2017. Asian-Australasian Journal of Animal Sciences, 31, 595-606; Zhou et al. 2018. Asian-Australasian Journal of Animal Sciences, 31, 32), were found altered upon ZEN exposure *in vivo.* However, the reproducibility and specificity of the studied parameters is limited, to the extent that no clear dose-response relationship between the increase/decrease of these biological parameters in blood, urine or bile and ZEN intake could be established so far. He et al. (He et al. 2018. Endocrinology, 159, 2993-3006) describe an increase of the expression of the microRNA (miRNA) miR-7 in pituitary tissue of ovariectomized pigs upon intraperitoneal injection of 112.5 mg ZEN per day. However, He et al. applied an experimental setup that does not reflect natural ZEN exposure due to contaminated feed, since the amount of 112.5 mg ZEN exceeds the realistic exposure level by far. Also, administration by injection does not reflect natural intake with the feed. Further, ovariectomized pigs are not good models for ZEN exposure of healthy, non-ovariectomized pigs, since ZEN exposure commonly correlates with reproduction and fertility disorders. Further, it would be desirable to identify a biomarker that would be useful for detection of ZEN exposure in a more accessible sample than pituitary tissue, such as serum.

The present invention was made in view of the prior art outlined above. In general, the inventors of the present application have found that mechanism-based biomarkers would be more appropriate for application in the field for detection of mycotoxicosis than biomarkers of exposure due to simpler detection methods and a direct correlation to mycotoxin effects in an individuum, but so far no suitable biomarker-of-effect or mechanism-based biomarker has been identified.

The present invention provides a method for detecting zearalenone (ZEN) effect, which method comprises determining in a test sample the expression level of at least one miRNA described herein; and comparing the expression level with the expression level of said at least one miRNA in a control sample. miRNAs (also referred to as microRNAs) are short RNA molecules of a length of commonly from about 19 to about 24 nucleotides. miRNAs are negative regulators of gene expression capable of blocking translation of mRNA into proteins or of degrading mRNA molecules. By providing a method for detecting ZEN effect comprising determining in a test sample the expression level of at least one miRNA described herein; and comparing the expression level with a reference value, the inventors have surprisingly identified a reliable way for detecting ZEN effect via a biomarker-of-effect which is suitable for application in the field.

The term "expression level" of a polynucleotide such as *e.g.* an mRNA molecule or a miRNA, refers to a relative or absolute amount of said polynucleotide. Expression levels of polynucleotides can be determined by appropriate strategies known to a person skilled in the art including but not limited to microarry experiments, quantitative real-time PCR, or so-called next-generation sequencing (NGS) technologies offered *e.g.* by Illumina, Pacific Biosciences, Oxford Nanopore Technologies etc.

Zearalenone (ZEN) is a nonsteroidal macrocyclic lactone with the following structural formula, which can be synthesized by way of the polyketide metabolic pathway:

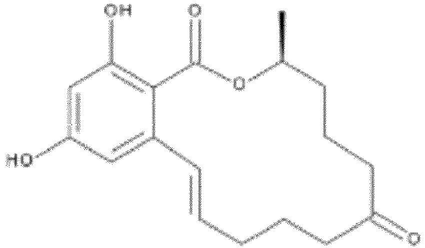

Its name according to the IUPAC nomenclature is (2E,11S)-15,17-dihydroxy-11-methyl-12-oxabicyclo[12.4.0]octadeca-1(18),2,14,16-tetraene-7,13-dione. A variety of ZEN derivatives also occurs in nature and may be formed by enzymatic or chemical modifications of ZEN. Examples include but are not limited to glycosidic ZEN conjugates or those containing sulfate, formed by fungi, plants or a mammalian metabolism as well as ZEN metabolites formed in the human or animal organism, among others. ZEN derivatives are understood below to be ZEN conjugates or ZEN metabolites that occur naturally or are synthesized by chemical or biochemical synthesis but in particular α-zearalenol (a-ZEL; (2E,7R,11 S)-7,15,17-trihydroxy-11-methyl-12-oxabicyclo[12.4.0]-octadeca-1(18),2,14,16-tetraen-13-one), β-zearalenol (β-ZEL; (2E,7S,11S)-7,15,17-trihydroxy-11-methyl-12-oxabicyclo[12.4.0]octadeca-1(18),2,14,16-tetraen-13-one), α-zearalanol (α-ZAL; (7R,11S)-7,15,17-trihydroxy-11-methyl-12-oxabicyclo[12.4.0]octadeca-1(18),14,16-trien-13-one), β-zearalanol (β-ZAL; (7S,11S)-7,15,17-trihydroxy-11-methyl-12-oxabicyclo[12.4.0]octadeca-1(14),15,17-trien-13-one), zearalenone 14-sulfate (Z14S; [(2E,11S)-15-hydroxy-11-methyl-7,13-dioxo-12-oxabicyclo[12.4.0]octadeca-1(18),2,14,16-tetraen-17-yl] hydrogen sulfate), zearalenone-14-glycoside (Z14G; (2E,11S)-15-hydroxy-11-methyl-17-[(3R,4S,5S,6R)-3,4,5-trihydroxy-6-(hydroxymethyl)-tetrahydropyran-2-yl]oxy-12-oxabicyclo[12.4.0]octadeca 1(18)2,14,16-tetraene-7,13-dione) as well as zearalanone (ZAN; (11S)-15,17-dihydroxy-11-methyl-12-oxabicyclo-[12.4.0]octadeca-1(18),14,16-triene-7,13-dione). ZEN as well as ZEN derivatives, in particular α-ZEL, β-ZEL, Z14S, α-ZAL, β-ZAL, Z14G and ZAN can also be detected in processed foods and animal feed products, such as bread or beer because of their high chemical and physical stability.

As used herein, the term "zearalenone effect" or "ZEN effect" refers to exposure of a biological system to zearalenone or a (physiological) effect of zearalenone on a biological system. The biological system may be any biological system, such as a cell, a cell culture, a tissue, or an animal. In some embodiments, "zearalenone effect" may further refer to exposure of a biological system to a zearalenone derivative or analog or a (physiological) effect of a zearalenone derivative or analog on a biological system. The zearalenone derivative is preferably one that is disclosed herein above. It is understood that the zearalenone derivative or analog is preferably one that has the same or a similar effect as zearalenone on the biological system. Preferably, "zearalenone effect" relates to zearalenone exposure.

A "test sample" as used herein preferably relates to a sample, for which it should be determined, whether or not it has been contacted to ZEN (or a ZEN analog or derivative) or whether the subject it has been obtained from has been exposed to ZEN (or a ZEN analog or derivative). The methods of the present invention may thus comprise step (b) providing the test sample.

The reference value may correspond to the expression level of said at least one miRNA according to the invention, that has been determined in one or more control samples. Depending on the type of the sample, such a control sample may have been contacted with (preferably a known amount of) ZEN or a ZEN analog or derivative, or a control sample that has not been contacted with ZEN or a ZEN analog or derivative. Such a control sample may have been obtained from a subject that has been exposed to (a preferably known amount of) ZEN or a ZEN analog or derivative, or may have been obtained from a subject that has not been exposed to ZEN or a ZEN analog or derivative. Here, contacting the sample with ZEN or a ZEN analog or derivative that is not bioavailable, e.g. because it is bound to another substance that prevents interaction of ZEN or a ZEN analog or derivative with a biological system, or exposing a subject to ZEN or a ZEN analog or derivative that is not bioavailable, is equivalent to not contacting ZEN or a ZEN analog or derivative with the sample or not exposing the subject with ZEN or a ZEN analog or derivative. The reference value may also be an average or mean value that has been determined in multiple control samples. The at least one miRNA according to the invention, may be selected from the group consisting of ssc-miR-1, ssc-miR-125a, ssc-miR-125b, ssc-miR-127, ssc-miR-129a, ssc-miR-133a-5p, ssc-miR-135, ssc-miR-136, ssc-miR-140-3p, ssc-miR-142-3p, ssc-miR-143-5p, ssc-miR-146b, ssc-miR-181c, ssc-miR-182, ssc-miR-183, ssc-miR-187, ssc-miR-195, ssc-miR-204, ssc-miR-206, ssc-miR-22-3p, ssc-miR-22-5p, ssc-miR-335, ssc-miR-34a, ssc-miR-369, ssc-miR-378, ssc-miR-424-5p, ssc-miR-432-5p, ssc-miR-450a, ssc-miR-450b-5p, ssc-miR-450c-5p, ssc-miR-455-3p, ssc-miR-455-5p, ssc-miR-486, ssc-miR-493-3p, ssc-miR-493-5p, ssc-miR-497, ssc-miR-503, ssc-miR-542-3p, ssc-miR-708-3p, ssc-miR-758, ssc-miR-7135-3p, hsa-miR-135b-5p, hsa-miR-301a-5p, put-miR-300. Preferably, the at least one miRNA according to the invention may be selected from the group consisting of ssc-miR-1, ssc-miR-181c, ssc-miR-206, ssc-miR-503, ssc-miR-542-3p, ssc-miR-135, ssc-miR-129a-3p, ssc-miR-142-3p, ssc-miR-432-5p, ssc-miR-455-5p, ssc-miR-182, ssc-miR-493-3p, ssc-miR-455-3p, ssc-miR-183, ssc-miR-140-3p, ssc-miR-7135-3p, ssc-miR-204, ssc-miR-143-5p, ssc-miR-187, ssc-miR-335, ssc-miR-424-5p, ssc-miR-450a, ssc-miR-450b-5p, ssc-miR-450c-5p, ssc-miR-497. The at least one miRNA of the invention may also be a combination of two, three, four, five, six, seven, eight, nine, ten, eleven, twelve, or even more, or all miRNAs selected from aforementioned group.

Preferably, the at least one miRNA according to the invention is selected from the group consisting of ssc-miR-1, ssc-miR-181c, ssc-miR-206, ssc-miR-503, ssc-miR-542-3p, ssc-miR-135, ssc-miR-129a-3p, ssc-miR-142-3p, ssc-miR-432-5p, ssc-miR-455-5p, ssc-miR-182, ssc-miR-493-3p, ssc-miR-455-3p, ssc-miR-183, and ssc-miR-140-3p. The at least one miRNA of the invention may also be a combination of two, three, four, five, six, seven, eight, nine, ten, eleven, twelve, thirteen, fourteen, or all fifteen miRNAs selected from aforementioned group. Hereby, zearalenone effect can be determined with particularly high reliability. Preferably, the at least one miRNA according to the invention is selected from the group consisting of ssc-miR-542-3p, ssc-miR-1, ssc-miR-493-3p, ssc-miR-135, ssc-miR-432-5p, and ssc-miR-455-5p. The at least one miRNA of the invention may also be a combination of two, three, four, five or all six miRNAs selected from aforementioned group. Preferred combinations of two miRNAs selected from aforementioned group comprise a pair selected from the group consisting of ssc-miR-542-3p and ssc-miR-1, ssc-miR-542-3p and ssc-miR-493-3p, ssc-miR-135 and ssc-miR-432-5p, and ssc-miR-455-5p and ssc-miR-493-3p. Preferred combinations may also comprise two, three, or four of the aforementioned pairs. Hereby, the accuracy of the method results can be increased.

Preferably, the at least one miRNA according to the invention is selected from the group consisting of ssc-miR-1, ssc-miR-181c, ssc-miR-206, ssc-miR-503, ssc-miR-542-3p, ssc-miR-135, ssc-miR-7135-3p, ssc-miR-204, ssc-miR-143-5p, ssc-miR-187, ssc-miR-335, ssc-miR-424-5p, ssc-miR-450a, ssc-miR-450b-5p, ssc-miR-450c-5p, and ssc-miR-497. The at least one miRNA of the invention may also be a combination of two, three, four, five, six, seven, eight, nine, ten, eleven, twelve, thirteen, fourteen, fifteen, or all sixteen miRNAs selected from aforementioned group. Preferably, the at least one miRNA according to the invention is ssc-miR-542-3p and/or ssc-miR-1.

Throughout the application, unless specified otherwise, miRNAs are denoted by accession numbers of the miRBase sequence database in the version of release 21 of June 2014 (Kozomara A, Griffiths-Jones S. Nucleic Acids Res. 2014 42:D68-D73; Kozomara A, Griffiths-Jones S. Nucleic Acids Res. 2011 39:D152-D157; Griffiths-Jones S, Saini HK, van Dongen S, Enright AJ. Nucleic Acids Res. 2008 36:D154-D158;Griffiths-Jones S, Grocock RJ, van Dongen S, Bateman A, Enright AJ. Nucleic Acids Res. 2006 34:D140-D144; Griffiths-Jones S. Nucleic Acids Res. 2004 32:D109-D111). The following table summarizes some of the miRNAs of the disclosure.

| **Accession No.** | **Sequence** | **SEQ ID NO** |
|---|---|---|
| ssc-miR-1 | uggaauguaaagaaguaugua | 01 |
| ssc-miR-125a | ucccugagacccuuuaaccugug | 02 |
| ssc-miR-125b | ucccugagacccuaacuuguga | 03 |
| ssc-miR-127 | ucggauccgucugagcuuggcu | 04 |
| ssc-miR-129a | aagcccuuaccccaaaaagcau | 05 |
| ssc-miR-133a-5p | agcugguaaaauggaaccaaau | 06 |
| ssc-miR-135 | uauggcuuuuuauuccuauguga | 07 |
| ssc-miR-136 | acuccauuuguuuugaugaugga | 08 |
| ssc-miR-140-3p | uaccacaggguagaaccacggac | 09 |
| ssc-miR-142-3p | uguaguguuuccuacuuuaugg | 10 |
| ssc-miR-143-5p | ggugcagugcugcaucucugg | 11 |
| ssc-miR-146b | ugagaacugaauuccauaggc | 12 |
| ssc-miR-181c | aacauucaaccugucggugagu | 13 |
| ssc-miR-182 | uuuggcaaugguagaacucacacu | 14 |
| ssc-miR-183 | uauggcacugguagaauucacug | 15 |
| ssc-miR-187 | ucgugucuuguguugcagccgg | 16 |
| ssc-miR-195 | uagcagcacagaaauauuggc | 17 |
| ssc-miR-204 | uucccuuugucauccuaugccu | 18 |
| ssc-miR-206 | uggaauguaaggaaguguguga | 19 |
| ssc-miR-22-3p | aagcugccaguugaagaacugu | 20 |
| ssc-miR-22-5p | aguucuucaguggcaagcuuua | 21 |
| ssc-miR-335 | ucaagagcaauaacgaaaaaug | 22 |
| ssc-miR-34a | uggcagugucuuagcugguugu | 23 |
| ssc-miR-369 | aauaauacaugguugaucuuu | 24 |
| ssc-miR-378 | acuggacuuggagucagaaggc | 25 |
| ssc-miR-424-5p | cagcagcaauucauguuuugaa | 26 |
| ssc-miR-432-5p | ucuuggaguaggucauugggu | 27 |
| ssc-miR-450a | uuuugcgauguguuccuaauau | 28 |
| ssc-miR-450b-5p | uuuugcaauauguuccugaaua | 29 |
| ssc-miR-450c-5p | uuuugcgauguguuccuaauac | 30 |
| ssc-miR-455-3p | gcaguccaugggcauauacac | 31 |
| ssc-miR-455-5p | uaugugccuuuggacuacaucg | 32 |
| ssc-miR-486 | uccuguacugagcugccccgag | 33 |
| ssc-miR-493-3p | ugaaggucuacugugugccagg | 34 |
| ssc-miR-493-5p | uuguacaugguaggcuuucauu | 35 |
| ssc-miR-497 | cagcagcacacugugguuugu | 36 |
| ssc-miR-503 | uagcagcgggaacaguacugcag | 37 |
| ssc-miR-542-3p | ugugacagauugauaacugaaa | 38 |
| ssc-miR-708-3p | caacuagacugugagcuucuaga | 39 |
| ssc-miR-758 | uuugugaccugguccacuaac | 40 |
| ssc-miR-7135-3p | aucugucugugucucugagcag | 41 |
| hsa-miR-135b-5p | uauggcuuuucauuccuauguga | 42 |
| hsa-miR-301a-5p | gcucugacuuuauugcacuacu | 43 |
| put-miR-300 | uugcaggaacuugugagucuccua | 44 |

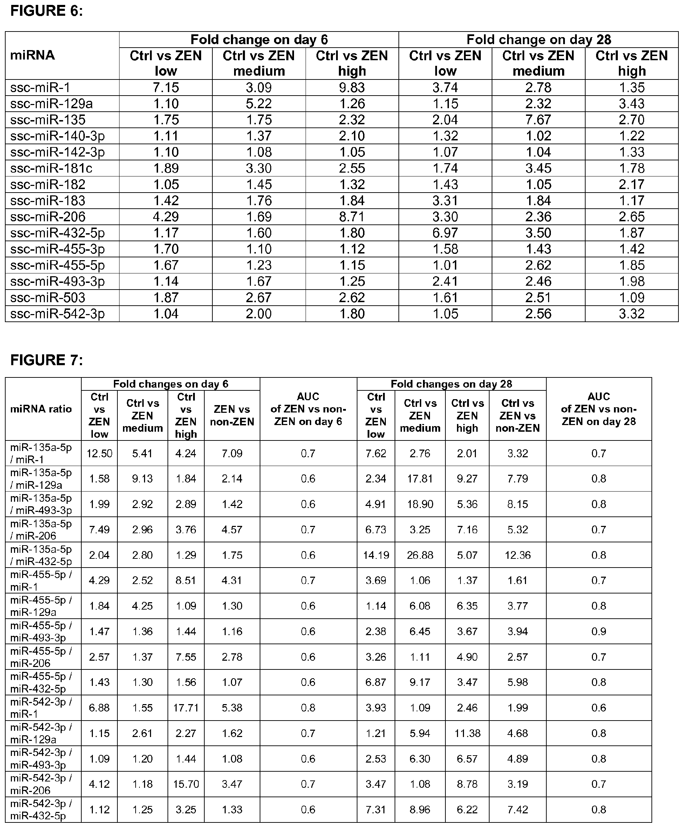

The methods of the present invention may comprise comparing the level of the at least one miRNA determined in a test sample with a reference value. If the at least one miRNA comprises more than one miRNA, comparing the level between these miRNAs may include comparing the ratio between two of the miRNAs. For example, where the at least one miRNA comprise two, three, four, or five miRNAs selected from the group consisting of or comprising at least some, preferably all of ssc-miR-542-3p, ssc-miR-1, ssc-miR-493-3p, ssc-miR-135, ssc-miR-432-5p, and ssc-miR-455-5p, comparing the level may refer to comparing the ratio between ssc-miR-542-3p and ssc-miR-1, ssc-miR-542-3p and ssc-miR-493-3p, ssc-miR-135 and ssc-miR-432-5p, and/or ssc-miR-455-5p and ssc-miR-493-3p. Comparing the level may also refer to comparing the ratio between any of the miRNA pairs shown in Figure 7.

Comparing the level of said at least one miRNA can be based on comparing the individual values of one or more miRNA. For example, a deviation in the expression levels of a miRNA between the test sample and reference value may be indicative for zearalenone effect (such as zearalenone exposure), in particular if the reference value corresponds to the expression level of said miRNA in a control sample that has not been contacted with zearalenone or that has been obtained from a subject that has not been exposed to zearalenone. A deviation in the expression levels may be indicative for zearalenone exposure. The deviation may be expressed in a normal scale, or preferably in a log-fold scale, such as log2-fold scale. The deviation is preferably statistically significant. Several techniques for testing the statistical significance of results are known to the skilled person and it is well within the skill of the person skilled in the art to choose and apply the most appropriate method for analyzing statistical significance. Statistical significance may e.g. be expressed by a p-value or a FDR p adjusted value and the significance level may be ≤0.07, preferably ≤0.05, or more preferably ≤0.001. The deviation may be an increase or decrease. A deviation indicating ZEN exposure may e.g. be at least about 1.3-fold, more preferably at least about 1.5-fold and most preferably at least about 2-fold compared to a reference value corresponding to a sample not having been contacted with zearalenone or obtained from a subject not having been exposed to zearalenone.

Alternatively, where the reference value corresponds to the expression level that has been determined in a control sample that has been contacted with ZEN or a ZEN analog or a ZEN derivative, or has been obtained from a subject that has been exposed to ZEN or a ZEN analog or a ZEN derivative, a non-deviation, a non-significant deviation, or an (untypically) low deviation between the values of test sample and the reference value may be indicative for zearalenone effect, such as zearalenone exposure.

The term "detect" or "detecting" when used herein in the context of detecting zearalenone effect may generally refer to quantitative or qualitative detection. For example, "detect" may refer to qualitative detection, i.e. whether or not a zearalenone effect is present based on the comparison step. "Detect" may also refer to a quantitative detection, i.e. to the extent of the zearalenone effect based on the comparison step.

A "sample" as used herein refers to a biological sample. The sample may comprise a cell. The sample may have been obtained from a subject. Biological samples include, but are not limited to, blood, serum, peripheral blood mononuclear cell (PBMC), urine, feces, semen, or tissue, such as uterine tissue. To allow a rapid detection of ZEN exposure in a subject and thus achieve maximum applicability in the field, a method for detection is preferably applicable to a sample that can be obtained with as little effort as possible. Therefore, the sample is preferably a blood sample, in particular a blood serum sample. By providing a method wherein the sample is a blood serum sample, potential ZEN exposure of a subject can be assessed most conveniently. A blood serum sample can be obtained *e.g.* by punctuation of the *Vena cava cranialis* or the *Vena jugalaris.* Serum samples can be stored at -80 °C for later analysis. A further preferred sample is a uterine sample. A sample may also be a cell culture sample. A cell culture sample may comprise intact cells, but may also comprise or consists of disrupted cells and/or supernatant of intact or disrupted cells. Cells comprised in the cell culture may express estrogen receptors α and/or β, preferably mammalian, such as human or porcine, estrogen receptors α and/or β. Preferred cell lines for a cell culture sample can be MCF-7, T47D, SUM159, HeLa or Ishikawa. Also, primary cells such as e.g. pig granulosa cells can be preferred cells for a cell culture sample.

Where the sample is a uterine tissue sample, the at least one miRNA is preferably selected from the group consisting of ssc-miR-1, ssc-miR-181c, ssc-miR-206, ssc-miR-503, ssc-miR-542-3p, ssc-miR-135, ssc-miR-7135-3p, ssc-miR-204, ssc-miR-143-5p, ssc-miR-187, ssc-miR-335, ssc-miR-424-5p, ssc-miR-450a, ssc-miR-450b-5p, ssc-miR-450c-5p, and ssc-miR-497, wherein the at least one miRNA is preferably ssc-miR-542-3p and/or ssc-miR-1.

Where the sample is a blood sample, such as a serum sample, the at least one miRNA may be selected from the group consisting of ssc-miR-1, ssc-miR-181c, ssc-miR-206, ssc-miR-503, ssc-miR-542-3p, ssc-miR-135, ssc-miR-129a-3p, ssc-miR-142-3p, ssc-miR-432-5p, ssc-miR-455-5p, ssc-miR-182, ssc-miR-493-3p, ssc-miR-455-3p, ssc-miR-183, and ssc-miR-140-3p, preferably selected from the group consisting of ssc-miR-542-3p, ssc-miR-1, ssc-miR-493-3p, ssc-miR-135, ssc-miR-432-5p, and ssc-miR-455-5p.

Comparing the level of said at least one miRNA can also be based on comparing the individual values of multiple miRNAs. Here the relative (or less preferred the absolute) deviations in the expression level in the test sample and the reference value may be combined to yield an even more significant result than comparing the level of only one miRNA. Alternatively, deviation values may be multiplied with or divided by each other, depending on the expected direction of deviation in case of a zearalenone effect.

Comparing the level of said at least one miRNA can also relate to comparison of the ratio of two miRNAs. The ratio may be determined by dividing the level of the one miRNA by the level of the second miRNA as known by those having skill in the art. The inventors found that the determination of the deviation of miRNA ratios as described before allowed surprisingly good discrimination between samples that were exposed to zearalenone (ZEN) and samples that were not exposed to zearalenone (ZEN). If individual measured miRNA are analyzed by qPCR, a possible way to calculate the ratio is by determining ΔCt (e.g. Ct miRNA - Ct UniSp4) for each miRNA to calculate the ratio. As an illustrative example, the ratio between miR-135a and miR-1a may be calculated by ΔCt miRNA 135a - ΔCt miRNA 1a.

Exceptionally good detection of ZEN effect can be achieved when the at least one ratio of expression levels between two miRNAs is selected from the group consisting of the ratios between miR-542-3p and miR-1, miR542-3p and miR493-3p, miR-135a-5p and miR-432-5p, and miR-455-5p and miR-493-3p. A ratio may be considered as being preferred for a method as described herein, if a receiver operating characteristics (ROC) analysis (Grund and Sabin. 2010. Current Opinion in HIV and AIDS, 5, 473-479) to discriminate ZEN exposure vs. non-ZEN exposure results in an area-under-the-curve (AUC)-value that is larger than or equal to 0.7. Alternatively or additionally, the fold change of a miRNA ratio in a ZEN-containing sample is preferably at least about 1.3-fold, more preferably at least about 1.5-fold, most preferably at least about 2-fold compared to a sample not contacted with ZEN or obtained from a subject not having been exposed to ZEN. The fold change can be either positive or negative, i.e. up- or down-regulated compared to a control sample. The level of at least one miRNA even more preferably comprises at least one ratio of expression levels between two miRNAs selected from the group consisting of miR-542-3p and miR-1, miR-542-3p and miR-493-3p, miR-135a-5p and miR-432-5p, and miR-455-5p and miR-493-3p. Hereby it is possible to detect ZEN exposure with especially good reliability and reproducibility.

Comparing the level of said at least one miRNA to a reference value, in particular if multiple miRNA are involved, can also be carried out using complex methods, such as computer-assisted methods, in particular methods involving machine learning. As an illustrative example, an artificial neural network system can be used for comparing the expression level(s) with a reference value. The neural network may be trained by being provided with the value of one or more reference values that may correspond to miRNA levels from one or more samples. Preferably, both, reference values for ZEN exposure and reference values for non-exposure to ZEN may be used for training the neural network. The trained neural network may then be used to detect zearalenone effect. For this purpose, the values of miRNA level(s) of respective miRNA(s) of a test sample may be used as input for the neural network, and the neural network may provide an indication of zearalenone effect, such as a qualitative information, a quantitative information, a score, a probability or any other type of information useful in the detection or characterization of a zearalenone effect. Methods of constructing, training, and applying a neural network are well known to the skilled person. Other (computer-assisted) approaches may also be suitable for being applied in the present invention and are also known the skilled person, such as e.g. Bayesian networks or decision trees.

Generally, the outcome of the comparing step (d) may be any type of information indicating whether or not the sample has been contacted with ZEN (or a ZEN analog or derivative) or whether or not the subject the sample has been obtained from has been exposed to ZEN (or a ZEN analog or derivative). The information may be a qualitative information, a quantitative information, a score, a probability or any other type of useful information. The method of the present invention may thus comprise the further step of (e) providing information regarding zearalenone effect. The information may be provided by any suitable mean known to the skilled person.

A "subject" as used herein refers to a vertebrate, preferably a mammal. The term "mammal" as used herein refers to any animal classified as a mammal, including, without limitation, humans, domestic and farm animals, and zoo, sports, or pet animals, such as sheep, dogs, horses, cats, cows, rats, pigs, primates such as cynomolgus monkeys, to name only a few illustrative examples. A preferred subject is of the genus *Sus,* preferably of the species *Sus scrofa.* Another preferred subject is a human.

ZEN effect, in particular exposure to ZEN, manifests itself as adverse health effects, such as reproduction and fertility disorders, especially in mammalian subjects and in a particularly dramatical manner in subjects of the genus *Sus.* To allow a most rapid detection of ZEN effect in said subjects, the present invention also relates to a method as described above, wherein the subject is preferably a mammal, more preferably of the genus *Sus.*

Methods of the present invention may be for diagnosis, in particular diagnosis of zearalenone exposure of the subject. Such methods may have the purpose of determining a medical condition of a subject in order to decide whether or not or what type of measure should be taken in order to maintain or improve the health of the subject.

Methods of the present invention may also be non-diagnostic. As an illustrative example, a non-diagnostic method can be a feed sampling method. Feed sampling is usually not very accurate due to the non-homogenous distribution of mycotoxins in the feed and typical sample sizes used for this analysis. Due to the comparably large amount of feed uptake by a subject, such as a pig, determining ZEN exposure of the subject may give a more reliable test result of whether or not the feed is contaminated with zearalenone than analyzing the feed directly. Methods of the present invention may therefore be methods for detection of zearalenone in feed or food. Methods of the present invention may comprise prior to obtaining the test sample from the subject step (a) feeding the subject with feed or food suspected to contain zearalenone.

To avoid the undesirable consequences of prolonged exposure to zearalenone, it may become necessary to treat ZEN contaminated feed or food with a ZEN neutralizing agent or to apply a ZEN neutralizing method, in case zearalenone effect is detected in a subject. Therefore, the present invention further relates to a method as described above, wherein the method is for selecting food or feed for contacting with a zearalenone neutralizing agent or for applying a zearalenone neutralizing method. By applying the method described for selecting food or feed for contacting with a ZEN neutralizing agent or for applying a ZEN neutralizing method, adverse health effects can be at least mitigated or completely avoided. Methods of the present invention may thus comprise step (a) feeding the subject with feed suspected to contain zearalenone prior to obtaining the test sample from the subject. Methods of the present invention may also comprise step (f) selecting the feed for contacting with a zearalenone neutralizing agent or applying a zearalenone neutralizing method if a deviation in the expression levels of the at least one miRNA between the test sample and the control sample indicates zearalenone exposure. Methods of the present invention may thus comprise providing a zearalenone neutralizing agent or applying a zearalenone neutralizing method. Methods of the present invention may also comprise contacting the feed or food selected by the method with the zearalenone neutralizing agent or applying the zearalenone neutralizing method to the feed.

A zearalenone neutralizing agent or method may refer to an agent or method that degrades zeralenone, e.g. by altering its molecular structure, which can be the effected by chemical, enzymatic, or physical interaction with the zearalenone neutralizing agent. A less toxic molecule may be the result of such a treatment. A zearalenone neutralizing agent or method may also reduce bioavailability of zearalenone, e.g. making zearalenone unavailable to the subject, preferably to the subject's digestion system. This may be achieved, for example by an agent that binds or adsorbs zearalenone. In general, a zearalenone neutralizing agent may be any suitable type of agent, which may be selected from the group consisting of one or more polypeptide(s), one or more microorganism(s), and one or more zearalenone-binding agent(s).

A zearalenone neutralizing agent may also be an α/β-hydrolase capable of transforming zearalenone or its derivatives or analogs. The person skilled in the art knows various α/β-hydrolases, which are *inter alia* described in Lenfant et al. (2013) 'ESTHER, the database of the α/β-hydrolase fold superfamily of proteins: tools to explore diversity of functions' Nucleic Acids Research, Volume 41, Issue D1, D423-D429 and Mindrebo et al. (2016) 'Unveiling the functional diversity of the Alpha-Beta hydrolase fold in plants' Curr Opin Struct Biol. 233-246. In short, all α/β-hydrolases share the feature of a specific fold called α/β-fold (alpha/beta-fold). The α/β- fold is common to a number of hydrolytic enzymes of widely differing phylogenetic origin and catalytic function. The core of each enzyme is an α/β-structure (rather than a barrel), containing 8 β-strands (b1-b8) connected by α-helices (aA-aF) (Ollis et al. (1992) 'The alpha/beta hydrolase fold' Protein Eng. 5(3):197-211). Therefore, an α/β-hydrolase as described herein can comprise an α/β-fold. An α/β-hydrolase as described herein preferably comprises the α/β-hydrolase core domain consisting of 8 β-strands (b1 - b8) arranged to a central β-sheet and additionally comprises 6 crossover α-helices (aA - aF).

Members of different classes of α/β-hydrolases as well as their structural characteristics are inter alia described in Kourist et al. (2010) 'The alpha/beta-hydrolase fold 3DM database (ABHDB) as a tool for protein engineering.' Chembiochem. 11(12):1635-43).

As enzymes, α/β-hydrolases are often described to be responsible for the hydrolysis of ester and peptide bonds. However, α/β-hydrolases also participate in the breaking of carbon-carbon bonds, decarboxylation reactions and cofactor-independent dioxygenation of heteroaromatic rings. Thus, α/β-hydrolases can include catalytic members (enzymes) in this superfamily. Non-limiting examples are hydrolases (acetylcholinesterase, carboxylesterase, dienelactone hydrolase, lipase, cutinase, thioesterase, serine carboxypeptidase, proline iminopeptidase, proline oligopeptidase, epoxide hydrolase) along with enzymes that require activation of HCN, H₂O₂ or O₂ instead of H₂O for the reaction mechanism (haloalkane dehalogenase, haloperoxidase, hydroxynitrile lyase). Non-catalytic members can include the neuroligins, glutactin, neurotactin, the C-terminal domain of thyroglobulin, yolk proteins, the CCG1-interacting-factor-B and dipeptidylaminopeptidase VI.

The ESTHER database gathers and annotates published information related to gene and protein sequences of this superfamily. Thus, the person skilled in the art can also obtain α/β-hydrolases from ESTHER (http://bioweb.supagro.inra.fr/ESTHER/general?what=index), a database of the α/β-hydrolase-fold superfamily of proteins.

An α/β-hydrolase as used herein may comprise the sequenceof SEQ ID NO: 45 or a sequence having 60% or more sequence identity to the sequence of SEQ ID NO: 45. Thus, any α/β-hydrolase comprising this sequence is embraced by the term α/β-hydrolase.

Additionally or alternatively, an α/β-hydrolase as used herein can also comprise the sequence of SEQ ID NO: 46 or a sequence having 60% or more sequence identity to the sequence of SEQ ID NO: 46. Thus, any α/β-hydrolase comprising this sequence is embraced by the term α/β-hydrolase.

Additionally or alternatively, an α/β-hydrolase as used herein can also comprise the sequence of SEQ ID NO: 47, 48 or 49 or a sequence having 60% or more sequence identity to the sequence of SEQ ID NO: 47, 48, or 49. Thus, any α/β-hydrolase comprising this sequence is embraced by the term α/β-hydrolase.

Additionally or alternatively, an α/β-hydrolase as used herein can also comprise the sequence of SEQ ID NO: 50 or a sequence having 60% or more sequence identity to the sequence of SEQ ID NO: 50. Thus, any α/β-hydrolase comprising this sequence is embraced by the term α/β-hydrolase.

For example, the α/β-hydrolase can comprise a sequence having at least 60 %, 65 %, 70 %, 75 %, 80 %, 85 %, 90 %, 95 %, 97 %, 98 %, 99 % identity to a sequence of SEQ ID NO: 45. Additionally or alternatively, the α/β-hydrolase can comprise a sequence having at least 60 %, 65 %, 70 %, 75 %, 80 %, 85 %, 90 %, 95 %, 97 %, 98 %, 99 % identity to a sequence of SEQ ID NO: 46. Additionally or alternatively, the α/β-hydrolase can comprise a sequence having at least 60 %, 65 %, 70 %, 75 %, 80 %, 85 %, 90 %, 95 %, 97 %, 98 %, 99 % identity to a sequence of SEQ ID NO: 47, 48, and/or 49. Additionally or alternatively, the α/β-hydrolase can comprise a sequence having at least 60 %, 65 %, 70 %, 75 %, 80 %, 85 %, 90 %, 95 %, 97 %, 98 %, 99 % identity to a sequence of SEQ ID NO: 50.

The sequences of SEQ ID NOs: 45-50 are shown in the Table below.

| SEQ ID NO | Sequence |
|---|---|
| 45 | MVTSPALRDVHVPHAYPEQQVDLGEITMNYAEAGDPDRPAVLLIPE QTGSWWSYEEAMGLLSEHFHVYAVDLRGQGRSSWTPKRYSLDNFG NDLVRFIALVVKRPVVVAGNSSGGVLAAWLSAYSMPGQLRGVLCE DPPFFASELVPAHGHSVRQGAGPVFELFRTYLGDQWSVGDWEGFCR AAGASASPMARSFVADGIPQHLQEYDPEWARVFYEGTVGLSCPHER MLGQVKTPVLLTHHMRGIDPETGNLLGALSDEQALRARRLMDSAG VTVDYESVPDASHMMHQSAPARYVEIFTRWAAALAP |
| 46 | MADPAQRDVYVPHAYPEKQADLGEITMNYAEAGEPDMPAVLLIPEQ TGSWWGYEEAMGLLAENFHVYAVDLRGQGRSSWAPKRYSLDNFG NDLVRFIALVVKRPVIVAGNSSGGVLAAWLSAYSMPGQVRGALCED |
| | APFFASELVTTCGHSIRQAAGPMFELFRTYLGDQWSVGDWTGYCRA ADASSSPMARYFVADEIPQHMREYDPEWARAFWEGTVALHCPHEQ LLTQVKTPVLLTHHMRDIDPDTGHLVGALSDEQAARARLLMESAGV KVDYASVPDALHMMHQFDPPRYVEIFTQWAATLAA |
| 47 | MVTSPALRDVHVPHAYPEQQVDLGEITMNYAEAGDPGRPAVLLIPE QTGSWWSYEEAMGLLAEHFHVYAVDLRGQGRSSWTPKRYSLDNFG NDLVRFIALVVRRPVVVAGNSSGGVLAAWLSAYSMPGQIRGVLCED PPFFASELVPAHGHSVRQGAGPVFELFRTYLGDQWSVGDWEGFRSA ADASASPMARSFVADTIPQHLKEYDPEWARAFYEGTVGLNCPHERM LNRVNTPVLLTHHMRGTDPETGNLLGALSDEQAAQVRRLMESAGV KVDYESVPDASHMMHQSDPARYAEILTPWTAALAP |
| 48 | MVTSPALRDVHVPHAYPEQQVDLGEITMNYAEAGDPDRPAVLLIPE QTGSWWSYEEAMGLLAEHFHVYAVDLRGQGRSSWTPKRYSLDNFG NDLVRFIALVVKRPVVVAGNSSGGVLAAWLSAYSMPGQLRGVLCE DPPFFASELVPAHGHSVRQGAGPVFELFRTYLGDQWSVSDWEGFCR AAGASASPMARSFVADGIPQHLKEYDPEWARAFHEGTVGLNCPHER MLGRVNTPVLLTHHMRGTDPETGNLLGALSDEQAAQARLLMESAG VRVDYESVPDASHMMHQSDPARYAEIFTRWAAALAP |
| 49 | MVTSPALRDVHVPHAYPEQQVDLGEITMNYAEAGDPGRPAVLLIPE QTGSWWSYEEAMGLLAEHFHVYAVDLRGQGRSSWTPKRYSLDNFG NDLVRFMALVVRRPVVVAGNSSGGVLAAWLSAYSMPGQIRGVLCE DPPFFASELVPAHGHSVRQGAGPVFELFRTYLGDQWSVGDWEGFRS AAGASASPMARSFVADTIPQHLKEYDPEWARAFYEGTVGLNCPHER MLNRVNTPVLLTHHMRGTDPETGNLLGALSDEQAAQARRLMESAG VKVDYESVPDASHMMHQSDPARYAEILTPWAAALAP |
| 50 | MAEEGTRSEAADAATQARQLPDSRNIFVSHRFPERQVDLGEVVMNF AEAGSPDNPALLLLPEQTGSWWSYEPVMGLLAENFHVFAVDIRGQG RSTWTPRRYSLDNFGNDLVRFIALVIKRPVVVAGNSSGGLLAAWLSA YAMPGQIRAALCEDAPFFASELVPAYGHSVLQAAGPAFELYRDFLG DQWSIGDWKGFVEAAKASPAKAMQLFPTPDEAPQNLKEYDPEWGR AFFEGTVALHCPHDRMLSQVKTPILITHHARTIDPETGELLGALSDLQ AEHAQDIIRSAGVRVDYQSHPDALHMMHLFDPARYAEILTSWSATLP |
| | AND |

The term "identical" or "percent identity" in the context of two or more polypeptide sequences such as SEQ ID NO: 45-50 refers to two or more sequences or subsequences that are the same, or that have a specified percentage of amino acids that are the same (e.g., at least 85 %, 90 %, 95 %, 96 %, 97 %, 98 % or 99 % identity), when compared and aligned for maximum correspondence over a window of comparison, or over a designated region as measured using a sequence comparison algorithm as known in the art, or by manual alignment and visual inspection. Sequences having, for example, 80 % to 95 % or greater sequence identity are considered to be substantially identical. Those having skill in the art will know how to determine percent identity between/among sequences using, for example, algorithms such as those based on CLUSTALW computer program (Thompson Nucl. Acids Res. 2 (1994), 4673-4680) or FASTDB (Brutlag Comp. App. Biosci. 6 (1990), 237-245), as known in the art.

Also available to those having skills in this art are the BLAST and BLAST 2.6 algorithms (Altschul Nucl. Acids Res. 25 (1977), 3389-3402). The BLASTP program for amino acid sequences uses as defaults a word size (W) of 6, an expect threshold of 10, and a comparison of both strands. Furthermore, the BLOSUM62 scoring matrix (Henikoff Proc. Natl. Acad. Sci., USA, 89, (1989), 10915; Henikoff and Henikoff (1992) 'Amino acid substitution matrices from protein blocks.' Proc Natl Acad Sci USA. 1992 Nov 15;89(22):10915-9) can be used.

For example, BLAST2.6, which stands for Basic Local Alignment Search Tool (Altschul, Nucl. Acids Res. 25 (1997), 3389-3402; Altschul, J. Mol. Evol. 36 (1993), 290-300; Altschul, J. Mol. Biol. 215 (1990), 403-410), can be used to search for local sequence alignments.

A zearalenone neutralizing agent may also be ozone as e.g. described in Qi L et al. Food Addit Contam Part A Chem Anal Control Expo Risk Assess. 2016 Nov;33(11):1700-1710. A zearalenone neutralizing agent may also be a microorganism, such as e.g. a *Clonostachys rosea* as described by Kosawang C et al., Fungal Biol. 2014 Apr;118(4):364-73, or a *Lactobacillus pentosus* as described by Arunrussamee Sangsila et al., Food Control, Volume 62, April 2016, Pages 187-192 or a yeast strain such as Trichosporon mycotoxinivorans MTV as described by Schatzmayr et al. Mycot. Res. 2003 19: 124-128. Further zearalenone neutralizing agents are e.g. described by Zinedine A et al., Food Chem Toxicol. 2007 Jan;45(1):1-18 or Ji C et al., Animal Nutrition Volume 2, Issue 3, 2016, Pages 127-133.

As described above, a zearalenone neutralizing agent may also reduce bioavailability of zearalenone e.g. by binding and/or adsorption. Examples for such agents can include cholestyramine, divinylbenzene-styrene, bentonites, montmorillonite-rich clays, zeolites, organoclays, activated charcoal, yeast or yeast cell wall-derived products.

Zearalenone neutralizing methods may comprise but are not limited to methods of contacting a zearalenone neutralizing agent with the feed. Methods may also comprise heat treatment or ultrasonic treatment.

The assessment of the efficacy and/or efficiency of mycotoxin neutralizing agents or mycotoxin neutralizing methods poses a considerable challenge due to the lack of suitable methods to allow a determination of whether or not the applied mycotoxin neutralizing agent or method achieved the desired the effect. Methods of the present invention may thus also serve the purpose of assessing the capacity of a method or (test) compound to neutralize ZEN. With other words a method of the invention may be for assessment of the capacity of a method or compound to neutralize ZEN. Such a compound may be selected from the group consisting of one or more polypeptide(s), one or more microorganism(s), including live, dead, lyophilized, autolyzed and/or dormant microorganism(s), and one or more zearalenone-binding agent(s), including organic and/or anorganic zearalenone-binding agent(s). By using the methods of the present invention, the assessment of the efficacy and/or efficiency of a ZEN neutralizing agent or method can be achieved in a particularly fast and quick manner.

In cases where a sample has been obtained from a subject, the method of the invention may thus comprise prior to obtaining the test sample from the subject step (a3) feeding the feed contacted with zearalenone and the test compound to the subject. In such a case, the method may further comprise steps of contacting the feed with zearalenone, contacting the feed with the test compound, and/or contacting zearalenone with the test compound. In general, such steps can be performed in any order. Accordingly the method may comprise prior to step (a3) steps of: (a1) contacting the feed with zearalenone, and (a2) contacting the feed contacted with zearalenone with the test compound; or (a1') contacting the feed with the test compound, and (a2') contacting the feed contacted with the test compound with zearalenone; or (a1'') contacting the zearalenone with the test compound, and (a2") contacting the zearalenone contacted with the test compound with the feed.

In some cases, which may be the case if the test sample is a cell culture, the sample may have been directly contacted with zearalenone and the test compound. The control sample used in such a method may be a negative control sample or positive control sample. In negative control sample used in this context refers to a sample that has been contacted with zearalenone and optionally a compound that does not neutralize zearalenone. A positive control sample used in this context refers to a sample that has been contacted with zearalenone and a compound that neutralizes zearalenone, or a sample that has not been contacted with zearalenone. In such a method, the absence of a deviation or a non-significant deviation in the expression levels between the test sample and the positive control sample may be indicative for the capacity of the test compound to neutralize zearalenone. Alternatively or additionally, the presence of a (preferably significant) deviation in the expression levels between the test sample and the negative control sample may be indicative for the capacity of the test compound to neutralize zearalenone. In such methods, the sample may be a sample that has not been obtained from a subject.

In order to avoid time-, labor- and cost-intensive animal trials, research experiments are preferrably performed in cultured cells. The test sample, positive control sample, and/or negative control sample may therefore be a cell culture. As an illustrative example, the cell culture may comprise MCF-7, T47D, SUM159, HeLa or Ishikawa. Also, primary cells such as e.g. pig granulosa cells may be comprised in the cell culture. By providing a method as described herein, wherein the test sample, the control sample, such as the positive control sample or the negative control sample is a cell culture, it is possible to obtain valuable data, *e.g.* on the capacity of a method or compound to neutralize ZEN, in a particularly efficient manner.

The present invention also relates to a use of at least one miRNA according to the inventon for detecting zearalenone effect in a test sample. The at least one miRNA may be selected from the group consisting of ssc-miR-1, ssc-miR-125a, ssc-miR-125b, ssc-miR-127, ssc-miR-129a, ssc-miR-133a-5p, ssc-miR-135, ssc-miR-136, ssc-miR-140-3p, ssc-miR-142-3p, ssc-miR-143-5p, ssc-miR-146b, ssc-miR-181c, ssc-miR-182, ssc-miR-183, ssc-miR-187, ssc-miR-195, ssc-miR-204, ssc-miR-206, ssc-miR-22-3p, ssc-miR-22-5p, ssc-miR-335, ssc-miR-34a, ssc-miR-369, ssc-miR-378, ssc-miR-424-5p, ssc-miR-432-5p, ssc-miR-450a, ssc-miR-450b-5p, ssc-miR-450c-5p, ssc-miR-455-3p, ssc-miR-455-5p, ssc-miR-486, ssc-miR-493-3p, ssc-miR-493-5p, ssc-miR-497, ssc-miR-503, ssc-miR-542-3p, ssc-miR-708-3p, ssc-miR-758, ssc-miR-7135-3p, hsa-miR-135b-5p, hsa-miR-301a-5p, put-miR-300. Preferably, the at least one miRNA according to the invention may be selected from the group consisting of ssc-miR-1, ssc-miR-181c, ssc-miR-206, ssc-miR-503, ssc-miR-542-3p, ssc-miR-135, ssc-miR-129a-3p, ssc-miR-142-3p, ssc-miR-432-5p, ssc-miR-455-5p, ssc-miR-182, ssc-miR-493-3p, ssc-miR-455-3p, ssc-miR-183, ssc-miR-140-3p, ssc-miR-7135-3p, ssc-miR-204, ssc-miR-143-5p, ssc-miR-187, ssc-miR-335, ssc-miR-424-5p, ssc-miR-450a, ssc-miR-450b-5p, ssc-miR-450c-5p, ssc-miR-497. The use may be in any method of the invention. Such a use may be for the purpose of detecting zearalenone exposure of a subject, assessing the capacity of a test compound or a method to neutralize zearalenone, or selecting a food for treatment with a zearalenone neutralizing agent or method. By using at least one miRNA as described herein for detecting zearalenone effect in a test sample, ZEN effect can be detected with especially high reliability and specificity.

To allow a rapid reaction such as *e.g.* the supply with a counteracting agent in case of ZEN exposure, fast availability of test results is desired. This can for example be achieved if determination of the expression level of at least one miRNA in a test sample and comparing the expression level with a reference value is performed with an integrated system. The methods of the invention may thus be computer-implemented methods, which may be carried out by an apparatus for carrying out such a method.

Thus, the present invention also relates to a data processing system comprising a processor configured to perform a method comprising the steps of comparing the expression level of at least one miRNA according to the invention determined in a test sample obtained from a subject with a reference value. Input regarding the expression level of the at least one miRNA is required. The information may be transmitted to the data processing system via a device that is capable of determining such information, e.g. over some transmission medium, such as over electrical wiring or cabling, through fiber optics, or via electromagnetic radiation. Such a device may be integrated in the data processing system or connected to it. Information regarding the expression level of the at least one miRNA may also be transmitted to the data processing system via input through a user interface. If the reference value corresponds to the expression level of said miRNA in a control sample that has been obtained from a subject that has not been exposed to zearalenone, a (significant) deviation in the expression levels of a miRNA between the test sample and reference value may be indicative for zearalenone effect (such as zearalenone exposure). In such a case the method may comprise indicating zearalenone exposure of the subject if a deviation is determined. Alternatively, if the reference value corresponds to the expression level that has been determined is a control sample that has been obtained from a subject that has been exposed to zearalenone, no deviation between the values of test sample and the reference value may be indicative for zearalenone effect, such as zearalenone exposure. In such a case the method may comprise indicating zearalenone exposure of the subject if no deviation or no significant deviation is determined. "No deviation" in this context may also encompass a non-significant deviation, or an (untypically) low deviation. The at least one miRNA may be selected from the group consisting of ssc-miR-1, ssc-miR-125a, ssc-miR-125b, ssc-miR-127, ssc-miR-129a, ssc-miR-133a-5p, ssc-miR-135, ssc-miR-136, ssc-miR-140-3p, ssc-miR-142-3p, ssc-miR-143-5p, ssc-miR-146b, ssc-miR-181c, ssc-miR-182, ssc-miR-183, ssc-miR-187, ssc-miR-195, ssc-miR-204, ssc-miR-206, ssc-miR-22-3p, ssc-miR-22-5p, ssc-miR-335, ssc-miR-34a, ssc-miR-369, ssc-miR-378, ssc-miR-424-5p, ssc-miR-432-5p, ssc-miR-450a, ssc-miR-450b-5p, ssc-miR-450c-5p, ssc-miR-455-3p, ssc-miR-455-5p, ssc-miR-486, ssc-miR-493-3p, ssc-miR-493-5p, ssc-miR-497, ssc-miR-503, ssc-miR-542-3p, ssc-miR-708-3p, ssc-miR-758, ssc-miR-7135-3p, hsa-miR-135b-5p, hsa-miR-301a-5p, put-miR-300. Preferably, the at least one miRNA according to the invention may be selected from the group consisting of ssc-miR-1, ssc-miR-181c, ssc-miR-206, ssc-miR-503, ssc-miR-542-3p, ssc-miR-135, ssc-miR-129a-3p, ssc-miR-142-3p, ssc-miR-432-5p, ssc-miR-455-5p, ssc-miR-182, ssc-miR-493-3p, ssc-miR-455-3p, ssc-miR-183, ssc-miR-140-3p, ssc-miR-7135-3p, ssc-miR-204, ssc-miR-143-5p, ssc-miR-187, ssc-miR-335, ssc-miR-424-5p, ssc-miR-450a, ssc-miR-450b-5p, ssc-miR-450c-5p, ssc-miR-497.

Ideally, the data processing system is connected to or integrated in a device that is capable of determining the expression level of at least one miRNA according to the invention in a test sample. The device may provide the data processing system with information regarding the expression level of said at least one miRNA. Such a device may for example be a sequencing device, such as a quantitative real-time sequencing device. Sequencing devices are known to the skilled person and are e.g. commercially available by Illumina, Pacific Biosciences, Oxford Nanopore Technologies etc. Particularly useful are portable sequencing devices such as a MinION sequencing (Oxford Nanopore Technologies). A device that is capable of determining the expression level of at least one miRNA according to the invention may also be a microarray reader.

Accordingly, the present invention also relates to a sequencing device capable of determining the level of at least one miRNA according to the present invetion, comprising the data processing system of the invention. The at least one miRNA may be selected from the group consisting of ssc-miR-1, ssc-miR-125a, ssc-miR-125b, ssc-miR-127, ssc-miR-129a, ssc-miR-133a-5p, ssc-miR-135, ssc-miR-136, ssc-miR-140-3p, ssc-miR-142-3p, ssc-miR-143-5p, ssc-miR-146b, ssc-miR-181c, ssc-miR-182, ssc-miR-183, ssc-miR-187, ssc-miR-195, ssc-miR-204, ssc-miR-206, ssc-miR-22-3p, ssc-miR-22-5p, ssc-miR-335, ssc-miR-34a, ssc-miR-369, ssc-miR-378, ssc-miR-424-5p, ssc-miR-432-5p, ssc-miR-450a, ssc-miR-450b-5p, ssc-miR-450c-5p, ssc-miR-455-3p, ssc-miR-455-5p, ssc-miR-486, ssc-miR-493-3p, ssc-miR-493-5p, ssc-miR-497, ssc-miR-503, ssc-miR-542-3p, ssc-miR-708-3p, ssc-miR-758, ssc-miR-7135-3p, hsa-miR-135b-5p, hsa-miR-301a-5p, put-miR-300. Preferably, the at least one miRNA according to the invention may be selected from the group consisting of ssc-miR-1, ssc-miR-181c, ssc-miR-206, ssc-miR-503, ssc-miR-542-3p, ssc-miR-135, ssc-miR-129a-3p, ssc-miR-142-3p, ssc-miR-432-5p, ssc-miR-455-5p, ssc-miR-182, ssc-miR-493-3p, ssc-miR-455-3p, ssc-miR-183, ssc-miR-140-3p, ssc-miR-7135-3p, ssc-miR-204, ssc-miR-143-5p, ssc-miR-187, ssc-miR-335, ssc-miR-424-5p, ssc-miR-450a, ssc-miR-450b-5p, ssc-miR-450c-5p, ssc-miR-497.

The present invention also relates to a computer program containing instructions to cause the data processing system disclosed herein or the sequencing device disclosed herein to execute the steps of (a) comparing the expression level of at least one miRNA according to the invention determined in a test sample obtained from a subject with a reference value and (b) indicating zearalenone exposure of the subject if a deviation is determined, wherein reference value corresponds to the expression level that has been determined in a control sample that has been obtained from a subject that has not been exposed to zearalenone; or indicating zearalenone exposure of the subject if no deviation is determined, wherein the reference value corresponds to the expression level that has been determined in a control sample that has been obtained from a subject that has been exposed to zearalenone. When the computer program is loaded into and executed by a data processing system, the data processing system becomes an apparatus for carrying out the methods described herein.

The computer program can be a computer program product accessible from a computer-usable or computer-readable medium providing program code for use by or in connection with a computer or any instruction execution system. The present invention thus also relates to a computer-readable medium having stored thereon the computer program disclosed herein. A computer-usable or computer readable medium can be any apparatus that may include, store, communicate, propagate, or transport the program for use by or in connection with the instruction execution system, apparatus, or device. The medium can be an electronic, magnetic, optical, electromagnetic, infrared, semiconductor, or paper system (or apparatus or device) or a propagation medium. The medium may be any suitable tangible medium, such as a diskette, a CD, a DVD, a BD, a hard drive, a memory stick, a memory card or any other computer readable storage medium.

In order to achieve particularly cost-effective and fast detection of ZEN exposure, a kit is provided for performing the method of detecting ZEN effect in the sense of the present invention, comprising a specific binding agent for at least one miRNA of the invention comprised on or conjugated to a solid support. The at least one miRNA may be selected from the group consisting of ssc-miR-1, ssc-miR-125a, ssc-miR-125b, ssc-miR-127, ssc-miR-129a, ssc-miR-133a-5p, ssc-miR-135, ssc-miR-136, ssc-miR-140-3p, ssc-miR-142-3p, ssc-miR-143-5p, ssc-miR-146b, ssc-miR-181c, ssc-miR-182, ssc-miR-183, ssc-miR-187, ssc-miR-195, ssc-miR-204, ssc-miR-206, ssc-miR-22-3p, ssc-miR-22-5p, ssc-miR-335, ssc-miR-34a, ssc-miR-369, ssc-miR-378, ssc-miR-424-5p, ssc-miR-432-5p, ssc-miR-450a, ssc-miR-450b-5p, ssc-miR-450c-5p, ssc-miR-455-3p, ssc-miR-455-5p, ssc-miR-486, ssc-miR-493-3p, ssc-miR-493-5p, ssc-miR-497, ssc-miR-503, ssc-miR-542-3p, ssc-miR-708-3p, ssc-miR-758, ssc-miR-7135-3p, hsa-miR-135b-5p, hsa-miR-301a-5p, put-miR-300. Preferably, the at least one miRNA according to the invention may be selected from the group consisting of ssc-miR-1, ssc-miR-181c, ssc-miR-206, ssc-miR-503, ssc-miR-542-3p, ssc-miR-135, ssc-miR-129a-3p, ssc-miR-142-3p, ssc-miR-432-5p, ssc-miR-455-5p, ssc-miR-182, ssc-miR-493-3p, ssc-miR-455-3p, ssc-miR-183, ssc-miR-140-3p, ssc-miR-7135-3p, ssc-miR-204, ssc-miR-143-5p, ssc-miR-187, ssc-miR-335, ssc-miR-424-5p, ssc-miR-450a, ssc-miR-450b-5p, ssc-miR-450c-5p, ssc-miR-497. The kit may comprise an unlabeled specific binding partner and a labeled specific binding partner for the at least one miRNA. Preferably, the unlabeled specific binding partner is conjugated to the solid support. The kit may comprise specific binding partners for no more than 1, 2, 3, 4, 5, 6, 7, 8, 9, 10, 11, 12, 13, 14, 15, 16, 17, 18, 19, 20, 21, 22, 23, 24, 25, or 26 miRNAs. In some applications, the kit may comprise specific binding partners for no more than 24, 48, 96, or 384 miRNAs. The kit may comprise specific binding partners for no more than 1, 2, 3, 4, 5, 6, 7, 8, 9, 10, 11, 12, 13, 14, 15, 16, 17, 18, 19, 20, 21, 22, 23, 24, 25, or 26 miRNAs according to methods of the invention. The kit may only comprise specific binding partners for miRNAs according to the methods of the invention.

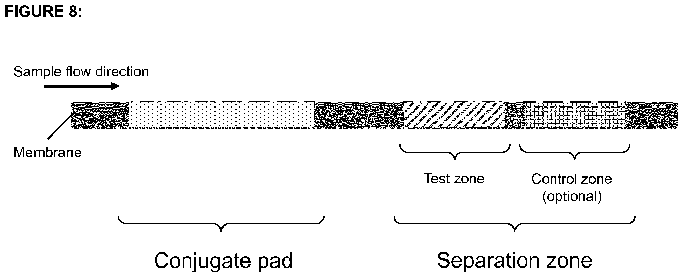

Such a kit may be for a lateral flow test. A schematic representation of a lateral flow device is shown in Fig. 8. The kit may comprise a lateral flow device (LFD), which comprises a carrier (solid support), which has at least two zones, a conjugate pad and a test zone. The conjugate pad comprises a labeled binding agent for the at least one miRNA according to the invention, that can be mobilized in a solvent (e.g. a solvent that comprises target molecule(s)) and be transported therein towards the test zone. The test zone comprises an un-labelled binding agent that is also capable of binding a target molecule. Hereby, targets (the at least one miRNA) that are also bound by the labelled agent from the conjugate pad are immobilized in the test zone. An optional additional control zone may comprise another immobilized binding agent, capable of binding the labelled binding agent from the conjugate pad which was not retained in the test zone. Examples of lateral flow devices are disclosed in Koczula and Gallotta. Essays Biochem. 2016 Jun 30;60(1):111-20 or Krska and Molinelli. Anal Bioanal Chem. 2009 Jan;393(1):67-71.

Also, immunochemical detection could be done by ELISA, e.g. Sandwich-ELISA. The kit may thus comprise a (preferably unlabeled) specific binding agent for the target miRNA that can be immobilized on a solid support or is immobilized on a solid support. The test sample may be added on to the support. Further, a labelled binding agent for the target miRNAs can be added. The miRNA-bound label may then be used for quantification of the miRNA.

The specific binding agent may for example be a nucleic acid, an antibody, an antigen binding fragment of an antibody, or a proteinaceous molecule having antibody-like properties. Where both, a labeled and an unlabeled specific binding partner, are used in the kit, it is preferred that both binding partners can simultaneously bind to the target miRNA. This may for example be achieved if one specific binding partner can bind (e.g. hybridize) to one part of the target miRNA, while the other specific binding partner can bind (e.g. hybridize) to another part of the target miRNA.

The kit may further comprise means for detecting a miRNA that is bound to a specific binding agent, such as an unlabeled specific binding agent described herein, which is preferably conjugated to a solid support. Such means may comprise a probe conjugated to a detectable label. For example, such means may be a labeled specific binding agent as described herein. In general, such a "detectable label" may be any appropriate chemical substance or enzyme, which directly or indirectly generates a detectable compound or signal in a chemical, physical, optical, or enzymatic reaction. For example, a fluorescent or radioactive label can be used to generate fluorescence or x-rays as detectable signal. A dye can be used to generate an optical signal. Alkaline phosphatase, horseradish peroxidase and β-galactosidase are examples of enzyme labels (and at the same time optical labels) which catalyze the formation of chromogenic reaction products. Preferably, the label does not negatively affect the characteristics of the binding partner to which the label is conjugated. A preferred detectable label is an optically detectable label, such as a chromophore or a fluorescent label.

The present invention is further characterized by the following items:
Item 1. A method for detecting zearalenone effect comprising: (c) determining in a test sample the expression level of at least one miRNA selected from the group consisting of ssc-miR-1, ssc-miR-181c, ssc-miR-206, ssc-miR-503, ssc-miR-542-3p, ssc-miR-135, ssc-miR-129a-3p, ssc-miR-142-3p, ssc-miR-432-5p, ssc-miR-455-5p, ssc-miR-182, ssc-miR-493-3p, ssc-miR-455-3p, ssc-miR-183, ssc-miR-140-3p, ssc-miR-7135-3p, ssc-miR-204, ssc-miR-143-5p, ssc-miR-187, ssc-miR-335, ssc-miR-424-5p, ssc-miR-450a, ssc-miR-450b-5p, ssc-miR-450c-5p, ssc-miR-497; and (d) comparing the expression level with a reference value.
Item 2. The method of item 1, wherein the reference value corresponds to the expression level of said at least one miRNA in a control sample.
Item 3. The method of item 1 or 2, wherein the zearalenone effect is zearalenone exposure.
Item 4. The method of any one of the preceding items, wherein the test sample is obtained from a subject.
Item 5. The method of any one of the preceding items, wherein a deviation in the expression level from the reference value is indicative for zearalenone exposure.
Item 6. The method of any one of the preceding items, wherein the deviation comprises an increase and/or decrease of the expression level of at least one of the miRNA according to item 1.
Item 7. The method of any one of the preceding items, wherein the deviation comprises an increase and/or decrease of at least one ratio of expression levels of at least two of the miRNA according to item 1.
Item 8. The method of any one of the preceding items, wherein the deviation is statistically significant.
Item 9. The method of any one of the preceding items, wherein the control sample has been obtained from a subject that has not been exposed to zearalenone or is a mean value of multiple samples that have been obtained from one or more subject(s) that has not been exposed to zearalenone.
Item 10. The method of any one of the preceding items wherein the sample is a blood sample or a tissue sample.
Item 11. The method of any one of the preceding items wherein the sample is a blood serum sample.
Item 12. The method of any one of the preceding items, wherein the at least one miRNA is selected from the group consisting of ssc-miR-1, ssc-miR-181c, ssc-miR-206, ssc-miR-503, ssc-miR-542-3p, ssc-miR-135, ssc-miR-129a-3p, ssc-miR-142-3p, ssc-miR-432-5p, ssc-miR-455-5p, ssc-miR-182, ssc-miR-493-3p, ssc-miR-455-3p, ssc-miR-183, and ssc-miR-140-3p.
Item 13. The method of item 11 or 12, wherein the at least one miRNA comprises two miRNAs selected from the group consisting of ssc-miR-542-3p and ssc-miR-1, ssc-miR-542-3p and ssc-miR-493-3p, ssc-miR-135 and ssc-miR-432-5p, and ssc-miR-455-5p and ssc-miR-493-3p.
Item 14. The method of any one of items 11 to 13, wherein the expression level of at least one miRNA comprises at least one ratio of expression levels between two miRNAs selected from the group consisting of ssc-miR-542-3p and ssc-miR-1, ssc-miR-542-3p and ssc-miR-493-3p, ssc-miR-135 and ssc-miR-432-5p, and ssc-miR-455-5p and ssc-miR-493-3p.
Item 15. The method of item 14, where in the at least one ratio of expression levels between two miRNAs is selected from the group consisting of the ratio between ssc-miR-542-3p and ssc-miR-1, ssc-miR-542-3p and ssc-miR-493-3p, ssc-miR-135 and ssc-miR-432-5p, and ssc-miR-455-5p and ssc-miR-493-3p.
Item 16. The method of any one of items 1 to 10, wherein the sample is a uterine tissue sample.
Item 17. The method of any one of the preceding items, wherein the at least one miRNA is selected from the group consisting of ssc-miR-1, ssc-miR-181c, ssc-miR-206, ssc-miR-503, ssc-miR-542-3p, ssc-miR-135, ssc-miR-7135-3p, ssc-miR-204, ssc-miR-143-5p, ssc-miR-187, ssc-miR-335, ssc-miR-424-5p, ssc-miR-450a, ssc-miR-450b-5p, ssc-miR-450c-5p, and ssc-miR-497.
Item 18. The method of item 16 or 17, wherein the at least one miRNA comprises miR-542-3p and miR-1.
Item 19. The method of item 18, wherein the expression level of at least one miRNA comprises the ratio of expression levels between miR-542-3p and miR-1.
Item 20. The method of any one of items 4 to 19, wherein the subject is a mammal.
Item 21. The method of any one of items 4 to 20, wherein the subject is of the genus *Sus.*
Item 22. The method of item 21, wherein the subject is of the species *Sus scrofa.*
Item 23. The method of any one of the preceding items further comprising (e) providing information regarding zearalenone effect.
Item 24. The method of any one of the preceding items further comprising (b) providing the test sample.
Item 25. The method of any one of the preceding items, wherein the method is for diagnosis.
Item 26. The method of any one of the preceding items, wherein the method is for diagnosis of zearalenone exposure.
Item 27. The method of any one of items 1 to 24, wherein the method is not for diagnosis.
Item 28. The method of any one of items 4 to 24 and 27, wherein the method is for detection of zearalenone in feed or food.
Item 29. The method of item 28, wherein the method comprises prior to obtaining the test sample from the subject (a) feeding the subject with feed suspected to contain zearalenone.
Item 30. The method of any one of items 4 to 24 and 27 to 29, wherein the method is for selecting feed for contacting with a zearalenone neutralizing agent or applying a zearalenone neutralizing method.
Item 31. The method of item 29 or 30, comprising (a) feeding the subject with feed suspected to contain zearalenone prior to obtaining the test sample from the subject; and/or (f) selecting the feed for contacting with a zearalenone neutralizing agent or applying a zearalenone neutralizing method if a deviation in the expression levels of the at least one miRNA between the test sample and the control sample indicates zearalenone exposure.
Item 32. The method of item 30 or 31, wherein the zearalenone neutralizing agent is selected from the group consisting of one or more polypeptide(s), one or more microorganism(s), and one or more zearalenone-binding agent(s).
Item 33. The method of any one of items 30 to 32, further comprising providing the zearalenone neutralizing agent.
Item 34. The method of any one of items 30 to 33, further comprising contacting the feed or food selected by the method with the zearalenone neutralizing agent or applying the zearalenone neutralizing method to the feed.
Item 35. The method of any one of items 4 to 22 and 25 to 27, wherein the method is for assessment of the capacity of a method or test compound to neutralize zearalenone.
Item 36. The method of item 35, wherein the test compound is selected from the group consisting of one or more polypeptide(s), one or more microorganism(s) and one or zearalenone-binding agent(s).
Item 37. The method of item 35 or 36, wherein the method comprises prior to obtaining the test sample from the subject (a3) feeding the feed contacted with zearalenone and the test compound to the subject.
Item 38. The method of item 37, wherein the method comprises prior to (a3) steps of: (a1) contacting the feed with zearalenone, and (a2) contacting the feed contacted with zearalenone with the test compound; or (a1') contacting the feed with the test compound, and (a2') contacting the feed contacted with the test compound with zearalenone; or (a1") contacting the zearalenone with the test compound, and (a2") contacting the zearalenone contacted with the test compound with the feed.
Item 39. The method of any one of items 1 to 3, wherein the method is for assessment of the capacity of a method or test compound to neutralize zearalenone.
Item 40. The method of item 39, wherein the test compound is selected from the group consisting of one or more polypeptide(s), one or more microorganism(s) and one or more zearalenone-binding agent(s).
Item 41. The method of item 39 or 40, wherein the test sample has been contacted with zearalenone and the test compound.
Item 42. The method of any one of items 39 to 41, wherein the control sample is a negative control sample or a positive control sample.
Item 43. The method of item 42, wherein the absence of a deviation in the expression levels between the test sample and the positive control sample or the presence of a deviation in the expression levels between the test sample and the negative control sample is indicative for the capacity of the test compound to neutralize zearalenone.
Item 44. The method of any one of items 40 to 43, wherein the test sample, the control sample, the positive control sample or the negative control sample is a cell culture.
Item 45. The method of item 30 to 44, wherein neutralization is by degradation.
Item 46. The method of item 30 to 44, wherein neutralization is by binding.
Item 47. Use of at least one miRNA selected from the group consisting of miR-1, miR-181c, miR-206, miR-503, miR-542-3p, miR-135, miR-135a-5p, miR-129a-3p, miR-142-3p, miR-432-5p, miR-455-5p, miR-182, miR-493-3p, miR-455-3p, miR-183, miR-140-3p, miR-7135-3p, miR-204, miR-143-5p, miR-187, miR-335, miR-424-5p, miR-450a, miR-450b-5p, miR-450c-5p, miR-497 for detecting zearalenone effect in a test sample.
Item 48. The use of item 47, wherein the use is for detecting zearalenone exposure of a subject.
Item 49. A data processing system comprising a processor configured to perform a method comprising the steps of (a) comparing the expression level of at least one miRNA selected from the group consisting of miR-1, miR-181c, miR-206, miR-503, miR-542-3p, miR-135, miR-135a-5p, miR-129a-3p, miR-142-3p, miR-432-5p, miR-455-5p, miR-182, miR-493-3p, miR-455-3p, miR-183, miR-140-3p, miR-7135-3p, miR-204, miR-143-5p, miR-187, miR-335, miR-424-5p, miR-450a, miR-450b-5p, miR-450c-5p, and miR-497 determined in a test sample obtained from a subject with a reference value; and (b) indicating zearalenone exposure of the subject if a deviation is determined, wherein reference value corresponds to the expression level that has been determined in a control sample that has been obtained from a subject that has not been exposed to zearalenone; or indicating zearalenone exposure of the subject if no deviation or is determined, wherein the reference value corresponds to the expression level that has been determined in a control sample that has been obtained from a subject that has been exposed to zearalenone.
Item 50. A sequencing device capable of determining the level of at least one miRNA selected from the group consisting of miR-1, miR-181c, miR-206, miR-503, miR-542-3p, miR-135, miR-135a-5p, miR-129a-3p, miR-142-3p, miR-432-5p, miR-455-5p, miR-182, miR-493-3p, miR-455-3p, miR-183, miR-140-3p, miR-7135-3p, miR-204, miR-143-5p, miR-187, miR-335, miR-424-5p, miR-450a, miR-450b-5p, miR-450c-5p, and miR-497, comprising the data processing system of item 49.
Item 51. A computer program comprising instructions to cause the data processing system of item 49 or the sequencing device of item 50 to execute the steps of (b) comparing the expression level of at least one miRNA selected from the group consisting of miR-1, miR-181c, miR-206, miR-503, miR-542-3p, miR-135, miR-135a-5p, miR-129a-3p, miR-142-3p, miR-432-5p, miR-455-5p, miR-182, miR-493-3p, miR-455-3p, miR-183, miR-140-3p, miR-7135-3p, miR-204, miR-143-5p, miR-187, miR-335, miR-424-5p, miR-450a, miR-450b-5p, miR-450c-5p, and miR-497, determined in a sample obtained from a subject, with a reference value; and (c) indicating zearalenone exposure of the subject if a deviation is determined, wherein reference value corresponds to the expression level that has been determined in a control sample that has been obtained from a subject that has not been exposed to zearalenone; or indicating zearalenone exposure of the subject if no deviation is determined, wherein the reference value corresponds to the expression level that has been determined in a control sample that has been obtained from a subject that has been exposed to zearalenone.
Item 52. A computer-readable medium having stored thereon the computer program of item 51.
Item 53. A kit for performing the method of any one of items 1 to 38 comprising a specific binding agent for at least one miRNA selected from the group consisting of miR-1, miR-181c, miR-206, miR-503, miR-542-3p, miR-135, miR-135a-5p, miR-129a-3p, miR-142-3p, miR-432-5p, miR-455-5p, miR-182, miR-493-3p, miR-455-3p, miR-183, miR-140-3p, miR-7135-3p, miR-204, miR-143-5p, miR-187, miR-335, miR-424-5p, miR-450a, miR-450b-5p, miR-450c-5p, and miR-497 comprised in or conjugated to a solid support.
Item 54. The kit of item 53, wherein the specific binding agent is a nucleic acid, an antibody, an antigen binding fragment of an antibody, or a proteinaceous molecule having antibody-like properties.
Item 55. The kit of item 53 or 54, further comprising means for detecting a miRNA that is bound to the specific binding agent.
Item 56. The kit of item 55, wherein the means for detecting a miRNA that is bound to the specific binding agent comprises a probe conjugated to a detectable label.
Item 57. The kit of item 56, wherein the detectable label is an optically detectable label.

It must be noted that as used herein, the singular forms "a", "an" and "the" include plural references and vice versa unless the context clearly indicates otherwise. Thus, for example, a reference to "a miRNA" or "a process" includes one or more of such miRNAs or processes, respectively, and a reference to "the process" includes equivalent steps and processes that could be modified or substituted known to those of ordinary skill in the art. Similarly, for example, a reference to "miRNAs" includes "a miRNA".

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "more than" includes the concrete number. For example, "more than 20" means ≥20.

Throughout this specification and the claims or items, unless the context requires otherwise, the word "comprise" and variations such as "comprises" and "comprising" will be understood to imply the inclusion of a stated integer (or step) or group of integers (or steps). It does not exclude any other integer (or step) or group of integers (or steps). When used herein, the term "comprising" can be substituted with "containing", "composed of', "including", "having" or "carrying."

The term "about" or "approximately" as used herein means within 20%, preferably within 10%, and more preferably within 5% of a given value or range. It includes, however, also the concrete number, e.g., about 20 includes 20.

When used herein "consisting of' excludes any element, step, or ingredient not specified in the claim element. When used herein, "consisting essentially of' does not exclude materials or steps that do not materially affect the basic and novel characteristics of the claim.

In each instance herein any of the terms "comprising", "consisting essentially of' and "consisting of' may be replaced with either of the other two terms. The possibility to replace terms with each other is not to be understood that these terms are necessarily synonymous.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

All publications cited throughout the text of this specification (including all patents, patent applications, scientific publications, manufacturer's specifications, instructions, etc.) are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

For further clarification of all aspects of the invention, the invention is described in the following by means of figures and examples. Therein:
**FIG. 1** shows the average vulva surface areas of the animals (n=6) of the four treatment groups over the time course of the experiment.
**FIG. 2** shows the number of differentially expressed miRNAs in the uterus samples of ZEN-exposed piglets (with adjusted p-values ≤0.07).
**FIG. 3A** **and** **B** show fold change values of *S. scrofa-*specific miRNAs in uterus tissue significantly affected (*i.e.* with FDR p adjusted value ≤0.07) in at least one ZEN group upon ZEN exposure. The fold change values can be either positive or negative, i.e. up- or down-regulated compared to a control sample.
**FIG. 3C-3E** show fold change values of *S. scrofa-*specific miRNAs in uterus tissue that were not significantly affected (*i.e.* with FDR p adjusted value >0.07). The fold change values can be either positive or negative, i.e. up- or down-regulated compared to a control sample.
**FIG. 4** shows log2-transformed fold changes and expression levels (measured as Tags Per Million, TPM) of putative and predicted miRNAs significantly increased or decreased (FDR p adjusted value ≤0.07) upon ZEN exposure in uterus tissue.
**FIG. 5** shows the results of targeted qPCR-based serum analysis on day 6 and day 28, comparing the Ctrl and ZEN high group. Only miRNAs with quantifiable levels in at least four individuals in each treatment group were considered.
**FIG. 6** shows fold change values of miRNAs from the targeted qPCR-based serum analysis. The fold change values can be either positive or negative, i.e. up- or down-regulated compared to a control sample.
**FIG. 7** shows fold change values of miRNA ratios from the targeted qPCR-based serum analyis of all three ZEN groups as well as a combined value of the three ZEN groups vs control (*i.e.* ZEN vs non-ZEN). Ratios were considered suitable when the area under the curve (AUC) value was ≥0.7 for at least one sample day. The fold change values can be either positive or negative, i.e. up- or down-regulated compared to a control sample.
**FIG. 8** shows a schematic representation of a lateral flow device.

### EXAMPLES

### Example 1: Animals and study design

Weaned female piglets (sow: Landrace x Large White, boar: Pietrain; 30 +/- 2 days old) were obtained from a local producer and individually tagged. Piglets were housed in slatted floor pens under controlled environmental conditions. After an acclimatization period of seven days, twenty-four female piglets were selected based on both body weight and vulva size, and allocated to one of four groups (6 animals/group). Piglets received either blank feed (negative control; Ctrl) or feed contaminated with ZEN in three increasing concentrations (ZEN low: 0.17 mg/kg diet; ZEN medium: 1.5 mg/kg diet; ZEN high: 4.6 mg/kg diet). During the 28-day experimental period, each treatment group was given free access to water and the assigned diet. The pigs were observed daily and weighed weekly.

Diets were manufactured at BIOMIN AN (Herzogenburg, Austria), and formulated according to the energy and amino acid requirements for piglets. Four different batches were prepared, one control batch and three batches artificially contaminated with ZEN (Fermentek LTD, Israel; purity > 99.2%). Homogenous distribution of ZEN within the diets was achieved by preparation of maltodextrin premixes, which contained appropriate amounts of ZEN and were added to the basal feed at an inclusion rate of 0.9% (w/w). Final ZEN concentrations were verified by HPLC-MS (Romer Labs GmbH) and confirmed.

Blood was collected weekly (d 0, 6, 14, 21 and 28) from individual animals by punctuation of the *vena cava cranialis* (Primavette® EDTA, Kabe Labortechnik GmbH, Nümbrecht-Elsenroth, Deutschland). After centrifugation (1500 x g, 20 min), serum samples were stored at -80 °C for later analysis. Vulva length and width were measured at regular intervals with a caliper to determine the dietary ZEN effects. The vulva area was calculated as vulva length × vulva width, as previously reported (Jiang et al. 2011. Journal of animal science, 89, 3008-3015). Eight measurements were done per piglet over the experimental period.

After 28 days of dietary exposure to the experimental diets, pigs were anesthetized by intramuscular injection of ketamine (0.1 mL/kg body weight (b.w.) Narketan®, Vetoquinol GmbH, Ravensburg, Germany) and azaperone (0.03 mL/kg b.w.; Stresnil®, Janssen-Cilag, Neuss, Germany). Subsequently, animals were euthanized by intracardial injection of T61® (Intervet, Unterschleißheim, Germany; 0.1 ml/kg b.w.). The whole reproductive tract was collected, weighed and examined for any clinical signs. Small pieces of the corpus uteri and the mid-jejunum were dissected, placed into 1 mL of RNAlater, and stored overnight at 4 °C. The next day, all the tissue samples were transferred to -80 °C for long-term storage, and until processed for transcriptomics analysis.

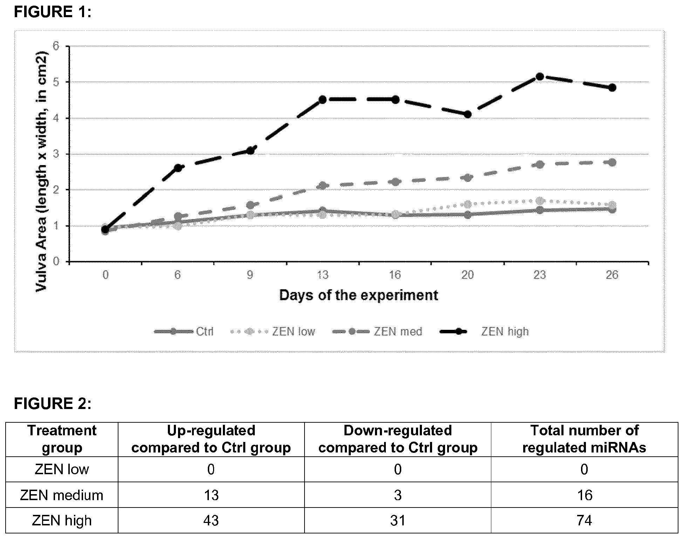

Results: Exposure to ZEN increased in a dose- and time-dependent manner the vulva surface (see FIG. 1). Specifically, piglets of the ZEN medium and ZEN high group showed a significant increase of the vulva surface after thirteen and six days of exposure, respectively. At d 26, the increase of vulva surface compared to the control group was +90% for ZEN medium and +230% for ZEN high. Similarly, the weight of the whole reproductive tract (including vagina, uterus, uterine tubes, ovaries) was significantly increased in piglets of the ZEN high group. The reproductive tract weight was expressed relative to the individual body weight ([reproductive tract (in grams)/body weight (in kg)]*100), and the calculated average (± SD) was as follows: 51.8 ± 20.6, 55.8 ± 17.2, 121.4 ± 43.4 and 353.4 ± 110.6 in the Control, ZEN low, ZEN medium and ZEN high group, respectively. Significant differences were observed between the ZEN high and control group (p-values <0.001). Independent of the concentration, ZEN exposure had no effect on the body weight of the piglets.

### Example 2: miRNA analysis in tissues

miRNA Extraction and Library Preparation: A total of 18 animals was used for investigations on the miRNA profile in the tissues: 6 from Ctrl, 4 from ZEN low, 4 from ZEN medium, and 4 from ZEN high. About 30 mg of tissue from uterus or jejunum was disrupted via a bead-beating step, and the total RNA, including RNA from approximately 18 nucleotides upwards, was extracted and purified with the miRNeasy Mini Kit (Qiagen, Hilden, Germany), following the manufacturer's instructions. The concentration of RNA was first estimated on the Nanodrop (Termo Scientifc NanoDrop 2000, USA), and again with the Agilent 2100 Bioanalyzer (Agilent Technologies, Germany) during the measurement of the RIN for determining the RNA quality. In total 1 µg of total RNA was used for construction of small RNA sequencing libraries using the NEBNext Multiplex SmallRNA Library preparation set for Illumina (New England Biolabs). Adapter-ligated total RNA was reverse transcribed into cDNA, which was used as template for PCR amplification (15 cycles) using Illumina's SR Primer and Index Primers 1-18. Barcoded DNA libraries were purified using the QIAQuick PCR purification protocol (Qiagen, Hilden, Germany) and quantified using the DNA 1000 high sensitivity chip (Agilent Technologies, Germany). Purified samples were pooled at equimolar (50 nM) concentration. Size selection was performed using gel purification to select for insert sizes between 18 and 50 nucleotides. The final multiplexed (n=18) libraries were sequenced on a NextSeq 500 sequencing instrument according to the manufacturer instructions. Raw data was de-multiplexed and FASTQ files for each sample was generated using the bcl2fastq software (Illumina inc.). FASTQ data was checked using the FastQC online tool (http://www.bioinformatics.babraham.ac.uk/projects/fastqc/).

RNA sequencing (RNA-Seq) Bioinformatics Analysis: Total reads were adapter trimmed using Cutadapt and quality filtered (Q-Score >30) using the FastQC tool. Trimmed and quality filtered reads were aligned to reference miRNA sequences in miRBase release 21 (www.mirbase.org; Kozomora and Griffiths-Jones. 2014. Nucleid Acids Research, 42, D68-D73) using bowtie2. Subsequently, reads were mapped against the *Sus scrofa* genome assembly Sscrofa10.2 as well as Ensembl small non-coding RNA reference databases. Reads were normalized to the total read count per library to generate tags per million (TPM) values. Clustvis was used for unsupervised clustering analysis. EdgeR (Robinson et al. 2010. Bioinformatics, 26, 139-140) was used for differential expression analysis based on TPM values. The p-values obtained were adjusted for multiple testing using Benjamini-Hochberg false discovery rate (FDR). Adjusted p-values ≤0.07 were considered significant.

RNA-Seq validation: Real-time polymerase chain reaction, also referred to as quantitative polymerase chain reaction (qPCR), was employed for verification of some results generated in the RNA-Seq for the uterus. Starting from the same total RNA samples used for sequencing analysis, cDNA was synthesized using the Universal cDNA Synthesis Kit II (Exiqon, Denmark) in a reverse transcription (RT) reaction using 50 ng of total RNA. Reaction conditions were used according to the manufacturer's recommendations. To monitor RT efficiency and presence of impurities with inhibitory activity, a synthetic RNA spike-in (cel-miR-39-3p) was added to the RT reaction. PCR amplification was performed in a 96-well plate format in a Roche LC480 II instrument (Roche, Germany) and using the EXiLENT SYBR Green mastermix (Exiqon, Denmark) with the following settings: 95 °C for 10 min, 45 cycles of 95 °C for 10 s and 60 °C for 60 s, followed by melting curve analysis. To calculate the cycle of quantification values (Cq-values), the second derivative method was used. Cq-values were normalized to the mean Cq-value in each sample (global mean normalization) by subtracting the individual miRNA Cq-value from the Cq average calculated for that sample.

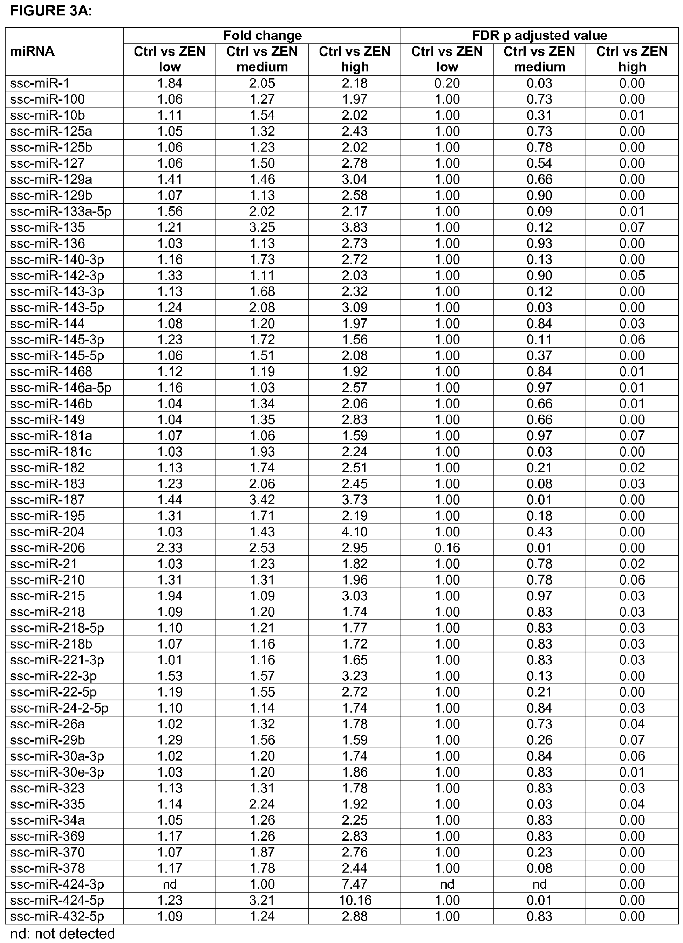

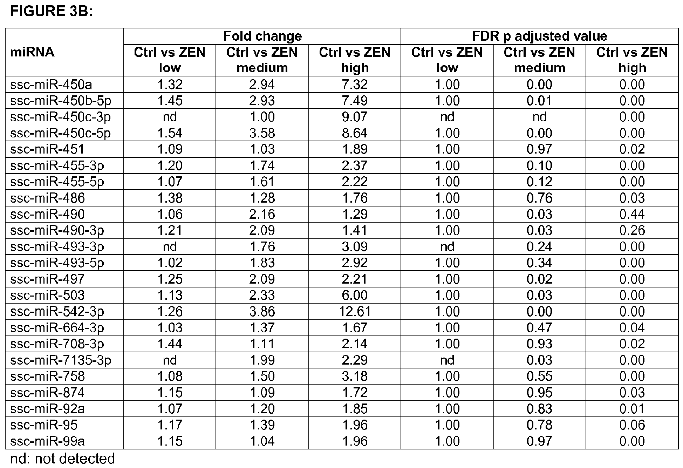

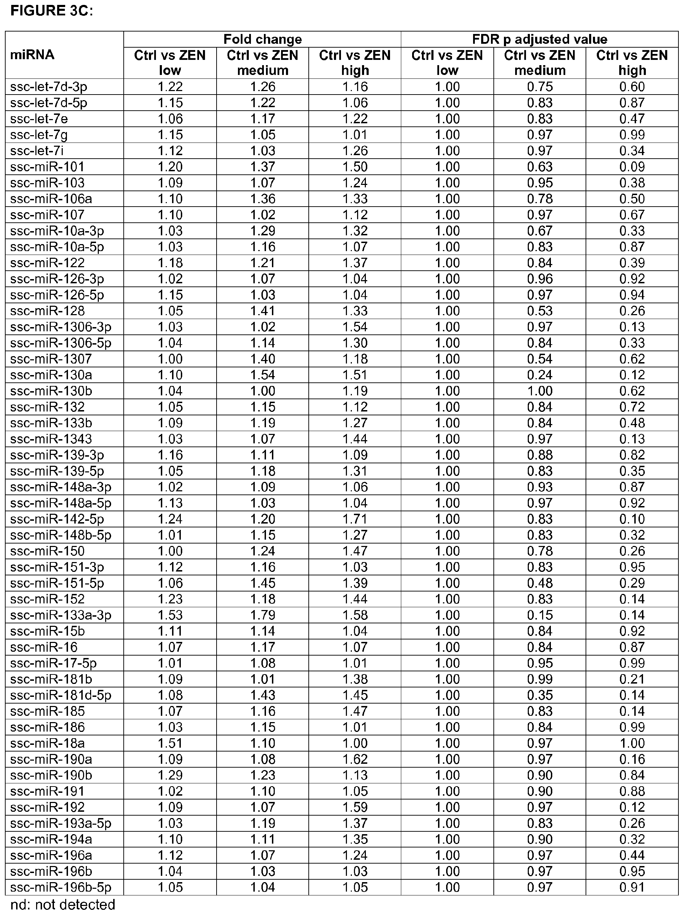

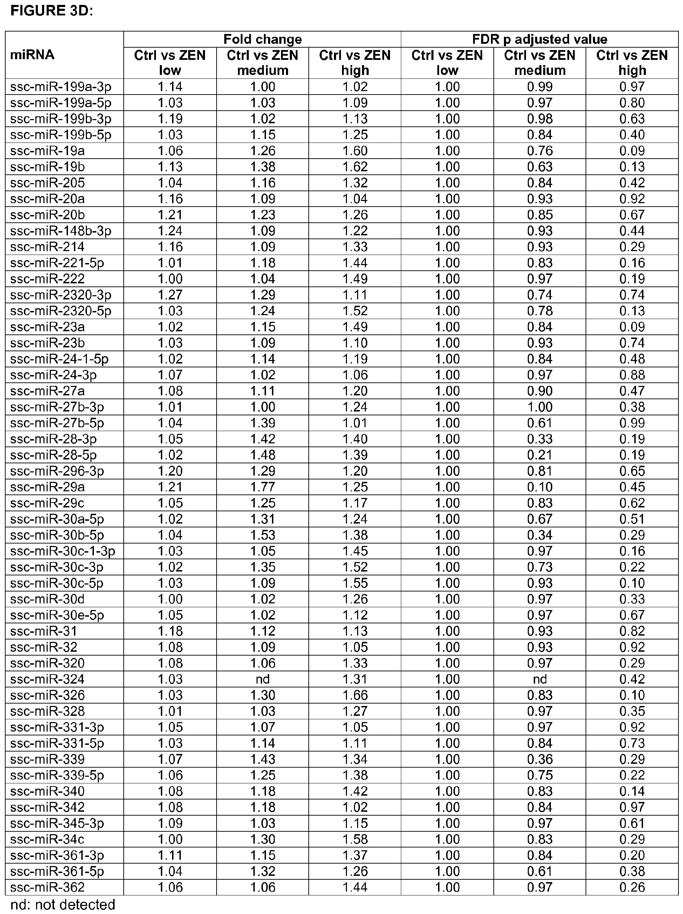

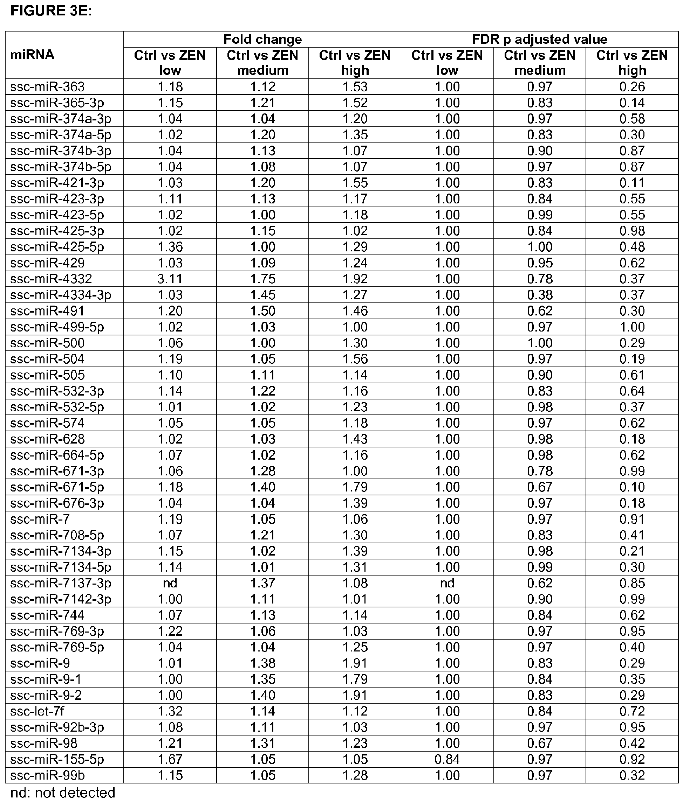

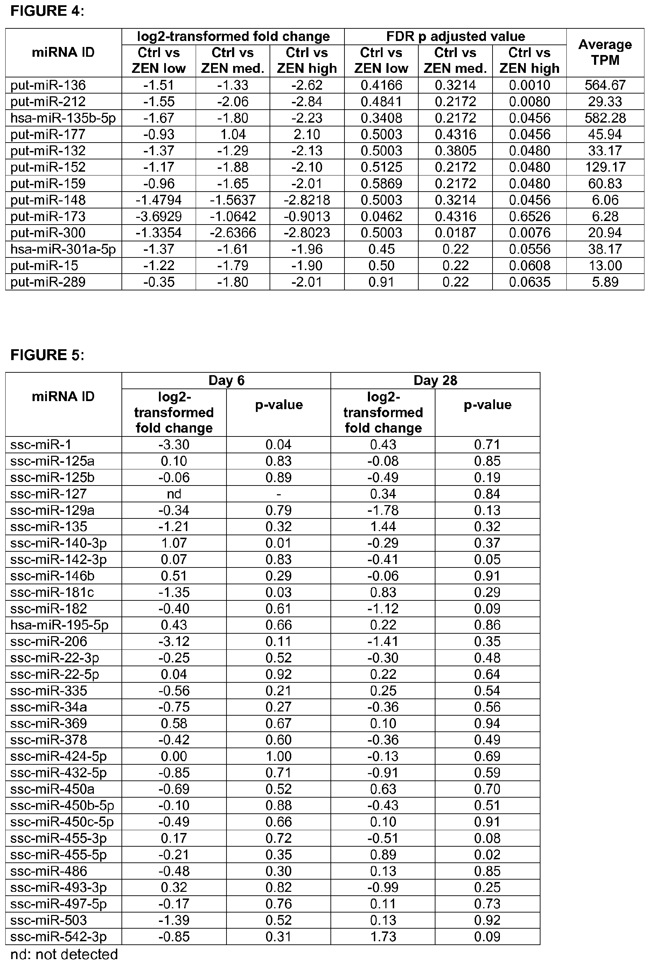

Results: Small RNA sequencing revealed the presence of 224 miRNAs (with tags per million > 1) known in pig species (ssc) in the uterus samples out of a total of 461 mature miRNAs that are currently known for pigs from miRBase. Out of those, 16 (ZEN medium) and 74 (ZEN high) miRNAs were significantly affected after ZEN exposure. FIG. 2 gives an overview on the number of up- and down-regulated miRNAs in the different treatment groups. In the ZEN medium group, fold changes of significantly affected species-specific miRNAs reached from -3.4 (ssc-miRNA-187) to +3.9 (ssc-miRNA-542-3p) compared to the control group (FIG. 3A and 3B). In the ZEN high group, effects were more pronounced with fold changes reaching from -4.1 (ssc-miRNA-204) to +12.6 (ssc-miRNA-542-3p). In total, 14 miRNAs (ssc-miR-1, ssc-miR-143-5p, ssc-miR-181c, ssc-miR-187, ssc-miR-206, ssc-miR-335, ssc-miR-424-5p, ssc-miR-450a, ssc-miR-450b-5p, ssc-miR-450c-5p, ssc-miR-497, ssc-miR-503, ssc-miR-542-3p, ssc-miR-7135-3p) were commonly affected in ZEN medium and ZEN high group. ZEN exposure led to a significant expression deviation of 13 putative or predicted miRNAs (FIG. 4). Out of those, put-miR-300 was significantly affected in ZEN medium and ZEN high. To validate the next-generation RNA sequencing results ssc-miR-542-3p, ssc-miR-450c-5p, ssc-miR-503, ssc-miR-424-5p, ssc-miR-181c were successfully quantified in uterus samples by qPCR for all groups.

### Example 3: miRNA analysis in blood serum samples

miRNA extraction and qPCR analysis: Unlike the small-RNA-Seq analysis, all the animals from the feeding trial were used for investigations of the miRNA profile in the serum (6 per group; total of 24 animals). RNA was isolated from the serum samples using the miRNeasy Mini Kit (Qiagen, Germany). Serum samples were thawed on ice and centrifuged at 12,000 x g for 5 minutes to remove any cellular debris. For each sample, 200 µL of serum was mixed with 1000 µL of Qiazol and 1 µL of synthetic spike-ins (Exiqon, Denmark). After a 10 minute incubation at room temperature, 200 µL chloroform was added to the lysates followed by cooled centrifugation at 12,000 x g for 15 minutes at 4 °C. A volume of 650 µL of the upper aqueous phase was mixed with 7 µL glycogen (50 mg/mL) to enhance precipitation. Samples were transferred to a miRNeasy mini column, and RNA was precipitated with 750 µL ethanol followed by washing with RPE and RWT buffer. RNA was eluted in 30 µL nuclease free water and stored at -80 °C until further analysis. Starting from total RNA samples, cDNA was synthesized using the Universal cDNA Synthesis Kit II (Exiqon, Denmark). Reaction conditions were chosen according to the recommendations of the manufacturer. In total 2 µL of purified total RNA was used per 10 µL reverse transcription (RT) reaction. To monitor RT efficiency and presence of impurities with inhibitory activity, a synthetic RNA spike-in (cel-miR-39-3p) was added to the RT reaction. PCR amplification was performed in a 384-well plate format using custom Pick&Mix plates (Exiqon, Denmark) in a Roche LC480 II instrument (Roche, Germany) and EXiLENT SYBR Green mastermix (Exiqon, Denmark) with the following settings: 95 °C for 10 min, 45 cycles of 95 °C for 10 s and 60 °C for 60 s, followed by melting curve analysis. To calculate the cycle of quantification values (Cq-values), the second derivative method was used, as known by a person skilled in the art. Cq-values were normalized to the mean Cq-value in each sample (global mean normalization) by subtracting the individual miRNA Cq-value from the Cq average calculated for that sample. The miRNAs selected for targeted analysis, their responses to ZEN treatment in the uterus samples and their relevances are shown in Table 1.

**Table 1: miRNAs selected for targeted analysis.**

| **miRNA ID** | **Response to ZEN treatment in uterus** | **Relevance** |
|---|---|---|
| ssc-miR-1 | Significantly increased in both ZEN medium and ZEN high group, coefficient of correlation > 0.8, log2 fold change Ctrl vs ZEN low ≥ 0.3, TPM > 10 | miRNA with significant dose-dependent effect and certain response in low dose group |
| ssc-miR-125a | Significantly decreased in ZEN high group, coefficient of correlation < 0.8, TPM > 10 | miRNA with significant effect in high dose group and relevant expression profile |
| ssc-miR-125b | Significantly decreased in ZEN high group, coefficient of correlation < -0.8, TPM > 10 | miRNA with significant effect in high dose group and relevant expression profile |
| ssc-miR-127 | Significantly increased in ZEN high group coefficient of correlation > 0.8, TPM > 10, specificity for reproductive tract | miRNA with significant effect in high dose group, relevant expression profile and certain tissue specificity |
| ssc-miR-129a | Significantly decreased in ZEN high group, coefficient of correlation < -0.8, log2 fold change Ctrl vs ZEN high < -1.5, specificity for reproductive tract | miRNA with significant effect in high dose group, relevant expression profile and certain tissue specificity |
| ssc-miR-133a-5p | Significantly increased in ZEN high group, coefficient of correlation > 0.8, log2 fold change Ctrl vs ZEN low ≥ 0.3, TPM > 10 | miRNA with significant effect in high dose group and certain response in low dose group |
| ssc-miR-135 | Significantly decreased in ZEN high group, log2 fold change change Ctrl vs ZEN high ≤ -1.5, TPM > 10 | miRNA with significant effect in high dose group and relevant expression profile |
| ssc-miR-136 | Significantly increased in ZEN high group, coefficient of correlation > 0.8, specificity for reproductive tract | miRNA with significant effect in high dose group, relevant expression profile and certain tissue specificity |
| ssc-miR-140-3p | Significantly increased in ZEN high group, coefficient of correlation > 0.8, TPM > 10 | miRNA with significant effect in high dose group and relevant expression profile |
| ssc-miR-142-3p | Significantly increased in ZEN high group, coefficient of correlation > 0.8, TPM > 10 | miRNA with significant effect in high dose group and relevant expression profile |
| ssc-miR-143-5p | Significantly increased in both ZEN medium and ZEN high group, coefficient of correlation > 0.8, log2 fold change Ctrl vs ZEN low ≥ 0.3, log2 fold change Ctrl vs ZEN high ≥-1.5, TPM > 10 | miRNA with significant dose-dependent effect and certain response in low dose group |
| ssc-miR-146b | Significantly decreased in ZEN high group, coefficient of correlation < -0.8, TPM > 10 | miRNA with significant effect in high dose group and relevant expression profile |
| ssc-miR-181 c | Significantly decreased in both ZEN medium and ZEN high group, coefficient of correlation > -0.8, TPM > 10 | miRNA with significant dose-dependent effect |
| ssc-miR-182 | Significantly increased in ZEN high group coefficient of correlation > 0.8, TPM > 10 | miRNA with significant effect in high dose group and relevant expression profile |
| ssc-miR-183 | Significantly increased in ZEN high group, coefficient of correlation > 0.8, TPM > 10 | miRNA with significant effect in high dose group and relevant expression profile |
| ssc-miR-187 | Significantly decreased in both ZEN medium and ZEN high group, coefficient of correlation > -0.8, log2 fold change Ctrl vs ZEN medium ≤ -0.3, log2 fold change Crtrl vs ZEN high ≤ - 15 | miRNA with effect in both ZEN medium and ZEN high, certain response in low dose group |
| ssc-miR-195 | Significantly increased in ZEN high group, coefficient of correlation > 0.8, log2 fold change Ctrl vs ZEN low ≥ 0.3, TPM > 10 | miRNA with significant effect in high dose group and certain response in low dose group |
| ssc-miR-204 | Significantly decreased in ZEN high group, coefficient of correlation < -0.8, log2 fold change Ctrl vs ZEN high ≤ - 1.5, TPM > 10, specificity for reproductive tract | miRNA with significant effect in high dose group, certain response in low dose group and tissue specificity |
| ssc-miR-206 | Significantly increased in both ZEN medium and ZEN high group, coefficient of correlation > 0.8, log2 fold change Ctrl vs ZEN low ≥ 0.3, log2 fold change, Ctrl vs ZEN high ≥ 1.5, TPM > 10, specificity for reproductive tract | miRNA with significant dose-dependent effect, certain response in low dose group and tissue specificity |
| ssc-miR-22-3p | Significantly increased in ZEN high group, coefficient of correlation > 0.8, log2 fold change Ctrl vs ZEN low ≥ 0.3, log2 fold Ctrl vs ZEN high ≥ 1.5; TPM > 10 | miRNA with significant effect in high dose group and certain response in low dose group |
| ssc-miR-22-5p | Significantly increased in ZEN high group, coefficient of correlation > 0.8 | miRNA with significant effect in high dose group and relevant expression profile |
| ssc-miR-335 | Significantly decreased in both ZEN medium and ZEN high group, TPM > 10 | miRNA with significant effect in both ZEN medium and ZEN high |
| ssc-miR-34a | Significantly increased in ZEN high group coefficient of correlation > 0.8 | miRNA with significant effect in high dose group and relevant expression profile |
| ssc-miR-369 | Significantly increased in ZEN high group coefficient of correlation > 0.8 | miRNA with significant effect in high dose group and relevant expression profile |
| ssc-miR-378 | Significantly increased in ZEN high group coefficient of correlation > 0.8, TPM > 10 | miRNA with significant effect in high dose group and relevant expression profile |
| ssc-miR-424-5p | Significantly increased in both ZEN medium and ZEN high group, coefficient of correlation > 0.8, log2 fold change Ctrl vs ZEN high ≥ 1.5, TPM > 10, specificity for reproductive tract | miRNA with significant dose-dependent effect, certain response in low dose group and certain tissue specificity |
| ssc-miR-432-5p | Significantly increased in ZEN high group, coefficient of correlation > 0.8, log2 fold change Ctrl vs ZEN high ≥ 1.5, specificity for reproductive tract | miRNA with significant effect in high dose group, relevant expression profile and certain tissue specificity |
| ssc-miR-450a | Significantly increased in both ZEN medium and ZEN high group, coefficient of correlation > 0.8, log2 fold change Ctrl vs ZEN high ≥ 1.5, TPM > 10, specificity for reproductive tract | miRNA with significant dose-dependent effect, certain response in low dose group and certain tissue specificity |
| ssc-miR-450b-5p | Significantly increased in both ZEN medium and ZEN high group, coefficient of correlation > 0.8, log2 fold change Ctrl vs ZEN high ≥ 1.5, TPM > 10, specificity for reproductive tract | miRNA with significant dose-dependent effect, certain response in low dose group and certain tissue specificity |
| ssc-miR-450c-5p | Significantly increased in both ZEN medium and ZEN high group, coefficient of correlation > 0.8, log2 fold change Ctrl vs ZEN high ≥ 1.5, TPM > 10, specificity for reproductive tract | miRNA with significant dose-dependent effect, certain response in low dose group and certain tissue specificity |
| ssc-miR-455-3p | Significantly increased in ZEN high group, coefficient of correlation > 0.8, TPM > 10 | miRNA with significant effect in high dose group and relevant expression profile |
| ssc-miR-455-5p | Significantly increased in ZEN high group, coefficient of correlation > 0.8, TPM > 10 | miRNA with significant effect in high dose group and relevant expression profile |
| ssc-miR-486 | Significantly increased in ZEN high group, coefficient of correlation > 0.8, log2 fold change Ctrl vs ZEN low ≥ 0.3, TPM > 10 | miRNA with significant effect in high dose group and certain response in low dose group |
| ssc-miR-493-3p | Significantly increased in ZEN high group, log2 fold change Ctrl vs ZEN high ≥ 1.5, specificity for reproductive tract | miRNA with significant effect in high dose group and relevant expression pattern |
| ssc-miR-493-5p | Significantly increased in ZEN high group, coefficient of correlation > 0.8, log2 fold change Ctrl vs ZEN low ≥ 0.3, TPM > 10, specificity for reproductive tract | miRNA with significant effect in high dose group, relevant expression profile and certain tissue specificity |
| ssc-miR-497 | Significantly increased in both ZEN medium and ZEN high group, log2 fold change Ctrl vs ZEN low ≥ 0.3, TPM > 10 | miRNA with significant effect in both ZEN medium and ZEN high, certain response in ZEN low |
| ssc-miR-503 | Significantly increased in both ZEN medium and ZEN high group, coefficient of correlation > 0.8, log2 fold change Ctrl vs ZEN high ≥ 1.5, TPM > 10, specificity for reproductive tract | miRNA with significant dose-dependent effect and certain tissue specificity |
| ssc-miR-542-3p | Significantly increased in both ZEN medium and ZEN high group, coefficient of correlation > 0.8, log2 fold change Ctrl vs ZEN low ≥ 0.3, log2 fold change Ctrl vs ZEN high ≥ 1.5, TPM > 10 | miRNA with significant dose-dependent effect and certain response in low dose group |
| ssc-miR-708-3p | Significantly decreased in ZEN high group, log2 fold change Ctrl vs ZEN low ≤ -0.3, TPM > 10 | miRNA with significant effect in high dose group and certain response in low dose group |
| ssc-miR-758 | Significantly increased in ZEN high group, coefficient of correlation > 0.8, log2 fold change Ctrl vs ZEN high ≥ 1.5 | miRNA with significant effect in high dose group and relevant expression profile |
| ssc-miR-7135-3p | Significantly increased in both ZEN medium and ZEN high group | miRNA with significant effect in both ZEN medium and ZEN high group |
| hsa-miR-135b-5p | Significantly decreased in ZEN high group, coefficient of correlation < -0.8; log2 fold change Ctrl vs ZEN low ≤ -0.3, log2 fold change Ctrl vs ZEN high ≤ -1.5, TPM > 10 | miRNA with significant effect in high dose group and certain response in low dose group |
| hsa-miR-301a-5p | Significantly decreased in ZEN high group, coefficient of correlation < -0.8, log2 fold change Ctrl vs ZEN low ≤ -0.3, log2 fold change Ctrl vs ZEN high ≤ -1.5, TPM > 10 | miRNA with significant effect in high dose group, relevant expression pattern and certain response in low dose group |
| put-miR-300 | Significantly decreased in ZEN medium and high group, log2 fold change Ctrl vs ZEN low ≤ -0.3, log2 fold change Ctrl vs ZEN high ≤ -1.5, TPM > 10 | miRNA in both ZEN medium and ZEN high group, relevant expression pattern and certain response in low dose group |

Results: Targeted analysis of ssc-miR-1, ssc-miR-125a, ssc-miR-125b, ssc-miR-127, ssc-miR-129a, ssc-miR-133a-5p, ssc-miR-135, ssc-miR-136, ssc-miR-140-3p, ssc-miR-142-3p, ssc-miR-143-5p, ssc-miR-146b, ssc-miR-181c, ssc-miR-182, ssc-miR-183, ssc-miR-187, ssc-miR-195, ssc-miR-204, ssc-miR-206, ssc-miR-22-3p, ssc-miR-22-5p, ssc-miR-335, ssc-miR-34a, ssc-miR-369, ssc-miR-378, ssc-miR-424-5p, ssc-miR-432-5p, ssc-miR-450a, ssc-miR-450b-5p, ssc-miR-450c-5p, ssc-miR-455-3p, ssc-miR-455-5p, ssc-miR-486, ssc-miR-493-3p, ssc-miR-493-5p, ssc-miR-497, ssc-miR-503, ssc-miR-542-3p, ssc-miR-708-3p, ssc-miR-758, ssc-miR-7135-3p, hsa-miR-135b-5p, hsa-miR-301a-5p, put-miR-300 was performed on serum samples collected after 6 and 28 days of ZEN exposure. For ssc-miR-135, ssc-miR-187, ssc-miR-195, ssc-miR-497, amplification via qPCR was done with primers designed for the human ortholog sequences. However, sequence identity between the pig and human sequence for these four miRNA was 100%. After removing several miRNAs from the dataset due to a too low read count (ssc-miR-143-5p, ssc-miR-187, ssc-miR-493-5p, ssc-miR-708-3p, ssc-miR-7135-3p, ssc-miR-135b-5p, hsa-miR-301a-5p, put-miR-300), focus was paid on the comparison of quantifiable miRNA levels between the Ctrl group and the ZEN high group. Three (day 6) and five (day 28) miRNAs showed up- or down-regulation after ZEN exposure (unadjusted p-value <0.1). Up-regulated miRNAs comprise ssc-miR-140-3p, ssc-miR-455-3p, ssc-miR-455-5p and ssc-miR-542-3p, while down-regulated miRNAs include ssc-miR-1, ssc-miR-142-3p, ssc-miR-181c and ssc-miR-182. FIG. 5 gives details on the log2-transformed fold changes of serum miRNA levels after ZEN exposure. In addition to the aforementioned miRNAs, ZEN had a considerable effect on log2-transformed fold changes (≤-0.8 or ≥0.8) of six miRNAs, namely ssc-miR-129a, ssc-miR-135, ssc-miR-206, ssc-miR-432-5p, ssc-miR-493-3p and ssc-miR-503. To further explore the global effects of ZEN regardless of the concentrations, principal component analyses (PCAs) were done on the data (fold changes) for all the groups and for all the miRNAs. This allowed detection of miRNAs which are not constant across all the treatments. Hereby some miRNAs were found that were already reported to be affected, but also new ones such as ssc-miR-183. Concluding from the data generated, ssc-miR-1, ssc-miR-129a, hsa-miR-135a-3p, ssc-140-3p, ssc-miR-142-3p, ssc-miR-181c, ssc-miR-182, ssc-miR-183, ssc-206, ssc-miR-432-5p, ssc-miR-455-3p, ssc-miR-455-5p, ssc-miR-493-3p, ssc-miR-503 and ssc-miR-542-3p were identified as suitable miRNAs to be used as mechanism-based biomarkers for ZEN effect, based on significant up- or down-regulation and relevant expression pattern which considers both relative fold change and absolute expression levels.

### Example 4: Correlation of miRNA levels in tissue and serum

To compare miRNA results obtained for uterus (NGS read count) and serum (qPCR dCq values), correlation analysis was performed. To this end, NGS read counts were log2-transformed and residuals (difference to mean) were calculated and used for correlation. Two miRNAs showed a trend for correlating tissue and serum expression levels: ssc-miR-455-5p (sample size: 17; correlation coefficient r=0.4045; significance level P=0.1073; 95% confidence interval for r: -0.09449 to 0.7411) and ssc-miR-542-3p (sample size: 17; correlation coefficient r=0.4244; significance level P=0.0895; 95% confidence interval for r: -0.07066 tot 0.7517).

### Example 5: miRNA ratios and Receiver Operating Characteristic (ROC) analysis

Ratios between selected miRNAs identified to be affected by ZEN exposure in the serum samples were established and subsequently subjected to the ROC (Receiver Operating Characteristic) approach as a validated and common method for evaluation of biomarkers (Grund and Sabin. 2010. Current Opinion in HIV and AIDS, 5, 473-479). Hereby, suitable ratios were identified that allowed discrimination between pigs non-exposed to ZEN (Ctrl) and pigs exposed to ZEN (ZEN low, ZEN medium, ZEN high): ssc-miR-135/ssc-miR-432-5p, ssc-miR-455-5p/ssc-miR-493-3p, ssc-miR-542-3p/ssc-miR-1 and ssc-miR-542-3p/ssc-miR-493-3p (FIG. 6).

In addition to the 15 serum miRNAs identified as suitable miRNAs to be used as mechanism-based biomarkers for ZEN effect described in Example 3, the miRNA ratios ssc-miR-135/ssc-miR-432-5p, ssc-miR-455-5p/ssc-miR-493-3p, ssc-miR-542-3p/ssc-miR-1 and ssc-miR-542-3p/ssc-miR-493-3p were found to be especially reliable and well-pronounced biomarkers to allow a detection of ZEN effect.

The invention illustratively described herein may suitably be practiced in the absence of any element or elements, limitation or limitations, not specifically disclosed herein. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by exemplary embodiments and optional features, modification and variation of the inventions embodied therein herein disclosed may be resorted to by those skilled in the art, and that such modifications and variations are considered to be within the scope of this invention.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the following claims. In addition, where features or aspects of the invention are described in terms of Markush groups, those skilled in the art will recognize that the invention is also thereby described in terms of any individual member or subgroup of members of the Markush group.

## Claims

1. A method for detecting zearalenone effect comprising:
(c) determining in a test sample the expression level of at least one miRNA selected from the group consisting of ssc-miR-1, ssc-miR-181c, ssc-miR-206, ssc-miR-503, ssc-miR-542-3p, ssc-miR-135, ssc-miR-129a-3p, ssc-miR-142-3p, ssc-miR-432-5p, ssc-miR-455-5p, ssc-miR-182, ssc-miR-493-3p, ssc-miR-455-3p, ssc-miR-183, ssc-miR-140-3p, ssc-miR-7135-3p, ssc-miR-204, ssc-miR-143-5p, ssc-miR-187, ssc-miR-335, ssc-miR-424-5p, ssc-miR-450a, ssc-miR-450b-5p, ssc-miR-450c-5p, ssc-miR-497; and
(d) comparing the expression level with a reference value.

2. The method of claim 1, wherein the reference value corresponds to the expression level of said at least one miRNA in a control sample.

3. The method of claim 1 or 2, wherein the at least one miRNA is selected from the group consisting of ssc-miR-1, ssc-miR-181c, ssc-miR-206, ssc-miR-503, ssc-miR-542-3p, ssc-miR-135, ssc-miR-129a-3p, ssc-miR-142-3p, ssc-miR-432-5p, ssc-miR-455-5p, ssc-miR-182, ssc-miR-493-3p, ssc-miR-455-3p, ssc-miR-183, and ssc-miR-140-3p.

4. The method of claim 3, wherein the expression level of at least one miRNA comprises at least one ratio of expression levels between two miRNAs selected from the group consisting of ssc-miR-542-3p and ssc-miR-1, ssc-miR-542-3p and ssc-miR-493-3p, ssc-miR-135 and ssc-miR-432-5p, and ssc-miR-455-5p and ssc-miR-493-3p.

5. The method of any one of the preceding claims, wherein the test sample has been obtained from a subject, wherein the subject is preferably a mammal, preferably of the genus *Sus,* preferably of the species *Sus scrofa.*

6. The method of any one of the preceding claims further comprising
(b) providing the test sample; and/or
(e) providing information regarding zearalenone effect.

7. The method of any one of the preceding claims, wherein the method is for diagnosis, preferably of zearalenone exposure.

8. The method of any one of claims 1 to 6, wherein the method is for detection of zearalenone in feed or food, wherein the method is preferably for selecting feed for contacting with a zearalenone neutralizing agent or applying a zearalenone neutralizing method.

9. The method of any one of claims 5-8, comprising
(a) feeding the subject with feed suspected to contain zearalenone prior to obtaining the test sample from the subject; and/or
(f) selecting the feed for contacting with a zearalenone neutralizing agent or applying a zearalenone neutralizing method if a deviation in the expression levels of the at least one miRNA between the test sample and the control sample indicates zearalenone exposure.

10. The method of any one of the preceding claims, wherein the method is for assessment of the capacity of a method or test compound to neutralize zearalenone.

11. Use of at least one miRNA selected from the group consisting of miR-1, miR-181c, miR-206, miR-503, miR-542-3p, miR-135, miR-135a-5p, miR-129a-3p, miR-142-3p, miR-432-5p, miR-455-5p, miR-182, miR-493-3p, miR-455-3p, miR-183, miR-140-3p, miR-7135-3p, miR-204, miR-143-5p, miR-187, miR-335, miR-424-5p, miR-450a, miR-450b-5p, miR-450c-5p, miR-497 for detecting zearalenone effect in a test sample.

12. A data processing system comprising a processor configured to perform a method comprising the steps of
(a) comparing the expression level of at least one miRNA selected from the group consisting of miR-1, miR-181c, miR-206, miR-503, miR-542-3p, miR-135, miR-135a-5p, miR-129a-3p, miR-142-3p, miR-432-5p, miR-455-5p, miR-182, miR-493-3p, miR-455-3p, miR-183, miR-140-3p, miR-7135-3p, miR-204, miR-143-5p, miR-187, miR-335, miR-424-5p, miR-450a, miR-450b-5p, miR-450c-5p, and miR-497 determined in a test sample obtained from a subject with a reference value; and
(b) indicating zearalenone exposure of the subject if a deviation is determined, wherein the reference value corresponds to the expression level that has been determined in a control sample that has been obtained from a subject that has not been exposed to zearalenone; or indicating zearalenone exposure of the subject if no deviation is determined, wherein the reference value corresponds to the expression level that has been determined in a control sample that has been obtained from a subject that has been exposed to zearalenone.

13. A sequencing device capable of determining the level of at least one miRNA selected from the group consisting of miR-1, miR-181c, miR-206, miR-503, miR-542-3p, miR-135, miR-135a-5p, miR-129a-3p, miR-142-3p, miR-432-5p, miR-455-5p, miR-182, miR-493-3p, miR-455-3p, miR-183, miR-140-3p, miR-7135-3p, miR-204, miR-143-5p, miR-187, miR-335, miR-424-5p, miR-450a, miR-450b-5p, miR-450c-5p, and miR-497, comprising the data processing system of claim 12.

14. A computer program comprising instructions to cause the data processing system of claim 12 or the sequencing device of claim 13 to execute the steps of
(b) comparing the expression level of at least one miRNA selected from the group consisting of miR-1, miR-181c, miR-206, miR-503, miR-542-3p, miR-135, miR-135a-5p, miR-129a-3p, miR-142-3p, miR-432-5p, miR-455-5p, miR-182, miR-493-3p, miR-455-3p, miR-183, miR-140-3p, miR-7135-3p, miR-204, miR-143-5p, miR-187, miR-335, miR-424-5p, miR-450a, miR-450b-5p, miR-450c-5p, and miR-497, determined in a sample obtained from a subject, with a reference value; and
(c) indicating zearalenone exposure of the subject if a deviation is determined, wherein the reference value corresponds to the expression level that has been determined in a control sample that has been obtained from a subject that has not been exposed to zearalenone; or indicating zearalenone exposure of the subject if no deviation is determined, wherein the reference value corresponds to the expression level that has been determined in a control sample that has been obtained from a subject that has been exposed to zearalenone; or
a computer-readable medium having stored thereon the computer program.

15. A kit for performing the method of any one of claims 1 to 10 comprising a specific binding agent for at least one miRNA selected from the group consisting of miR-1, miR-181c, miR-206, miR-503, miR-542-3p, miR-135, miR-135a-5p, miR-129a-3p, miR-142-3p, miR-432-5p, miR-455-5p, miR-182, miR-493-3p, miR-455-3p, miR-183, miR-140-3p, miR-7135-3p, miR-204, miR-143-5p, miR-187, miR-335, miR-424-5p, miR-450a, miR-450b-5p, miR-450c-5p, and miR-497, wherein the specific binding agent is comprised in or conjugated to a solid support.


SEQUENCE LISTING

<110> Erber Aktiengesellschaft

<120> Biomarker

<130> ERB16374EP

<160> 50

<170> BiSSAP 1.3.6

<210> 1
<211> 21
<212> RNA
<213> Sus scrofa

<220>
<223> microRNA

<400> 1
uggaauguaa agaaguaugu a                                                21

<210> 2
<211> 23
<212> RNA
<213> Sus scrofa

<220>
<223> microRNA

<400> 2
ucccugagac ccuuuaaccu gug                                              23

<210> 3
<211> 22
<212> RNA
<213> Sus scrofa

<220>
<223> microRNA

<400> 3
ucccugagac ccuaacuugu ga                                               22

<210> 4
<211> 22
<212> RNA
<213> Sus scrofa

<220>
<223> microRNA

<400> 4
ucggauccgu cugagcuugg cu                                               22

<210> 5
<211> 22
<212> RNA
<213> Sus scrofa

<220>
<223> microRNA

<400> 5
aagcccuuac cccaaaaagc au                                                22

<210> 6
<211> 22
<212> RNA
<213> Sus scrofa

<220>
<223> microRNA

<400> 6
agcugguaaa auggaaccaa au                                                22

<210> 7
<211> 23
<212> RNA
<213> Sus scrofa

<220>
<223> microRNA

<400> 7
uauggcuuuu uauuccuaug uga                                               23

<210> 8
<211> 23
<212> RNA
<213> Sus scrofa

<220>
<223> microRNA

<400> 8
acuccauuug uuuugaugau gga                                               23

<210> 9
<211> 23
<212> RNA
<213> Sus scrofa

<220>
<223> microRNA

<400> 9

uaccacaggg uagaaccacg gac                                              23

<210> 10
<211> 22
<212> RNA
<213> Sus scrofa

<220>
<223> microRNA

<400> 10
uguaguguuu ccuacuuuau gg                                               22

<210> 11
<211> 21
<212> RNA
<213> Sus scrofa

<220>
<223> microRNA

<400> 11
ggugcagugc ugcaucucug g                                                21

<210> 12
<211> 21
<212> RNA
<213> Sus scrofa

<220>
<223> microRNA

<400> 12
ugagaacuga auuccauagg c                                                21

<210> 13
<211> 22
<212> RNA
<213> Sus scrofa

<220>
<223> microRNA

<400> 13
aacauucaac cugucgguga gu                                               22

<210> 14
<211> 24
<212> RNA
<213> Sus scrofa

<220>
<223> microRNA

<400> 14
uuuggcaaug guagaacuca cacu                                              24


<210> 15
<211> 23
<212> RNA
<213> Sus scrofa


<220>
<223> microRNA

<400> 15
uauggcacug guagaauuca cug                                               23


<210> 16
<211> 22
<212> RNA
<213> Sus scrofa


<220>
<223> microRNA

<400> 16
ucgugucuug uguugcagcc gg                                                22


<210> 17
<211> 21
<212> RNA
<213> Sus scrofa


<220>
<223> microRNA

<400> 17
uagcagcaca gaaauauugg c                                                 21


<210> 18
<211> 22
<212> RNA
<213> Sus scrofa


<220>
<223> microRNA

<400> 18
uucccuuugu cauccuaugc cu                                                22


<210> 19
<211> 22
<212> RNA
<213> Sus scrofa

<220>
<223> microRNA

<400> 19
uggaauguaa ggaagugugu ga                                                22


<210> 20
<211> 22
<212> RNA
<213> Sus scrofa


<220>
<223> microRNA

<400> 20
aagcugccag uugaagaacu gu                                                22


<210> 21
<211> 22
<212> RNA
<213> Sus scrofa


<220>
<223> microRNA

<400> 21
aguucuucag uggcaagcuu ua                                                22


<210> 22
<211> 22
<212> RNA
<213> Sus scrofa


<220>
<223> microRNA

<400> 22
ucaagagcaa uaacgaaaaa ug                                                22


<210> 23
<211> 22
<212> RNA
<213> Sus scrofa


<220>
<223> microRNA

<400> 23
uggcaguguc uuagcugguu gu                                                22


<210> 24
<211> 21
<212> RNA
<213> Sus scrofa

<400> 24
aauaauacau gguugaucuu u                                                21

<210> 25
<211> 22
<212> RNA
<213> Sus scrofa

<220>
<223> microRNA

<400> 25
acuggacuug gagucagaag gc                                               22

<210> 26
<211> 22
<212> RNA
<213> Sus scrofa

<220>
<223> microRNA

<400> 26
cagcagcaau ucauguuuug aa                                               22

<210> 27
<211> 21
<212> RNA
<213> Sus scrofa

<220>
<223> microRNA

<400> 27
ucuuggagua ggucauuggg u                                                21

<210> 28
<211> 22
<212> RNA
<213> Sus scrofa

<220>
<223> microRNA

<400> 28
uuuugcgaug uguuccuaau au                                               22

<210> 29
<211> 22
<212> RNA
<213> Sus scrofa

<220>
<223> microRNA

<400> 29
uuuugcaaua uguuccugaa ua                                                22


<210> 30
<211> 22
<212> RNA
<213> Sus scrofa


<220>
<223> microRNA

<400> 30
uuuugcgaug uguuccuaau ac                                                22


<210> 31
<211> 21
<212> RNA
<213> Sus scrofa


<220>
<223> microRNA

<400> 31
gcaguccaug ggcauauaca c                                                 21


<210> 32
<211> 22
<212> RNA
<213> Sus scrofa


<220>
<223> microRNA

<400> 32
uaugugccuu uggacuacau cg                                                22


<210> 33
<211> 22
<212> RNA
<213> Sus scrofa


<220>
<223> microRNA

<400> 33
uccuguacug agcugccccg ag                                                22


<210> 34
<211> 22
<212> RNA

<213> Sus scrofa

<220>
<223> microRNA

<400> 34
ugaaggucua cugugugcca gg                                                22

<210> 35
<211> 22
<212> RNA
<213> Sus scrofa

<220>
<223> microRNA

<400> 35
uuguacaugg uaggcuuuca uu                                                22

<210> 36
<211> 21
<212> RNA
<213> Sus scrofa

<220>
<223> microRNA

<400> 36
cagcagcaca cugugguuug u                                                 21

<210> 37
<211> 23
<212> RNA
<213> Sus scrofa

<220>
<223> microRNA

<400> 37
uagcagcggg aacaguacug cag                                               23

<210> 38
<211> 22
<212> RNA
<213> Sus scrofa

<220>
<223> microRNA

<400> 38
ugugacagau ugauaacuga aa                                                22

<210> 39

<211> 23
<212> RNA
<213> Sus scrofa

<220>
<223> microRNA

<400> 39
caacuagacu gugagcuucu aga                                               23

<210> 40
<211> 21
<212> RNA
<213> Sus scrofa

<220>
<223> microRNA

<400> 40
uuugugaccu gguccacuaa c                                                 21

<210> 41
<211> 22
<212> RNA
<213> Sus scrofa

<220>
<223> microRNA

<400> 41
aucugucugu gucucugagc ag                                                22

<210> 42
<211> 23
<212> RNA
<213> Sus scrofa

<220>
<223> microRNA

<400> 42
uauggcuuuu cauuccuaug uga                                               23

<210> 43
<211> 22
<212> RNA
<213> Sus scrofa

<220>
<223> microRNA

<400> 43
gcucugacuu uauugcacua cu                                                22

<210> 44
<211> 24
<212> RNA
<213> Sus scrofa

<220>
<223> microRNA

<400> 44
uugcaggaac uugugagucu ccua                                              24

<210> 45
<211> 309
<212> PRT
<213> Artificial Sequence

<220>
<223> Alpha/beta hydrolase domain

<400> 45
Met Val Thr Ser Pro Ala Leu Arg Asp Val His Val Pro His Ala Tyr
1               5                   10                  15
Pro Glu Gln Gln Val Asp Leu Gly Glu Ile Thr Met Asn Tyr Ala Glu
            20                  25                  30
Ala Gly Asp Pro Asp Arg Pro Ala Val Leu Leu Ile Pro Glu Gln Thr
        35                  40                  45
Gly Ser Trp Trp Ser Tyr Glu Glu Ala Met Gly Leu Leu Ser Glu His
    50                  55                  60
Phe His Val Tyr Ala Val Asp Leu Arg Gly Gln Gly Arg Ser Ser Trp
65                  70                  75                  80
Thr Pro Lys Arg Tyr Ser Leu Asp Asn Phe Gly Asn Asp Leu Val Arg
                85                  90                  95
Phe Ile Ala Leu Val Val Lys Arg Pro Val Val Val Ala Gly Asn Ser
            100                 105                 110
Ser Gly Gly Val Leu Ala Ala Trp Leu Ser Ala Tyr Ser Met Pro Gly
        115                 120                 125
Gln Leu Arg Gly Val Leu Cys Glu Asp Pro Pro Phe Phe Ala Ser Glu
    130                 135                 140
Leu Val Pro Ala His Gly His Ser Val Arg Gln Gly Ala Gly Pro Val
145                 150                 155                 160
Phe Glu Leu Phe Arg Thr Tyr Leu Gly Asp Gln Trp Ser Val Gly Asp
                165                 170                 175
Trp Glu Gly Phe Cys Arg Ala Ala Gly Ala Ser Ala Ser Pro Met Ala
            180                 185                 190
Arg Ser Phe Val Ala Asp Gly Ile Pro Gln His Leu Gln Glu Tyr Asp
        195                 200                 205
Pro Glu Trp Ala Arg Val Phe Tyr Glu Gly Thr Val Gly Leu Ser Cys
    210                 215                 220
Pro His Glu Arg Met Leu Gly Gln Val Lys Thr Pro Val Leu Leu Thr
225                 230                 235                 240
His His Met Arg Gly Ile Asp Pro Glu Thr Gly Asn Leu Leu Gly Ala
                245                 250                 255
Leu Ser Asp Glu Gln Ala Leu Arg Ala Arg Arg Leu Met Asp Ser Ala
            260                 265                 270
Gly Val Thr Val Asp Tyr Glu Ser Val Pro Asp Ala Ser His Met Met
        275                 280                 285
His Gln Ser Ala Pro Ala Arg Tyr Val Glu Ile Phe Thr Arg Trp Ala
    290                 295                 300
Ala Ala Leu Ala Pro

305

<210> 46
<211> 308
<212> PRT
<213> Artificial Sequence

<220>
<223> Alpha/beta hydrolase domain

<400> 46
Met Ala Asp Pro Ala Gln Arg Asp Val Tyr Val Pro His Ala Tyr Pro
1               5                   10                  15
Glu Lys Gln Ala Asp Leu Gly Glu Ile Thr Met Asn Tyr Ala Glu Ala
            20                  25                  30
Gly Glu Pro Asp Met Pro Ala Val Leu Leu Ile Pro Glu Gln Thr Gly
        35                  40                  45
Ser Trp Trp Gly Tyr Glu Glu Ala Met Gly Leu Leu Ala Glu Asn Phe
    50                  55                  60
His Val Tyr Ala Val Asp Leu Arg Gly Gln Gly Arg Ser Ser Trp Ala
65                  70                  75                  80
Pro Lys Arg Tyr Ser Leu Asp Asn Phe Gly Asn Asp Leu Val Arg Phe
                85                  90                  95
Ile Ala Leu Val Val Lys Arg Pro Val Ile Val Ala Gly Asn Ser Ser
            100                 105                 110
Gly Gly Val Leu Ala Ala Trp Leu Ser Ala Tyr Ser Met Pro Gly Gln
        115                 120                 125
Val Arg Gly Ala Leu Cys Glu Asp Ala Pro Phe Phe Ala Ser Glu Leu
    130                 135                 140
Val Thr Thr Cys Gly His Ser Ile Arg Gln Ala Ala Gly Pro Met Phe
145                 150                 155                 160
Glu Leu Phe Arg Thr Tyr Leu Gly Asp Gln Trp Ser Val Gly Asp Trp
                165                 170                 175
Thr Gly Tyr Cys Arg Ala Ala Asp Ala Ser Ser Ser Pro Met Ala Arg
            180                 185                 190
Tyr Phe Val Ala Asp Glu Ile Pro Gln His Met Arg Glu Tyr Asp Pro
        195                 200                 205
Glu Trp Ala Arg Ala Phe Trp Glu Gly Thr Val Ala Leu His Cys Pro
    210                 215                 220
His Glu Gln Leu Leu Thr Gln Val Lys Thr Pro Val Leu Leu Thr His
225                 230                 235                 240
His Met Arg Asp Ile Asp Pro Asp Thr Gly His Leu Val Gly Ala Leu
                245                 250                 255
Ser Asp Glu Gln Ala Ala Arg Ala Arg Leu Leu Met Glu Ser Ala Gly
            260                 265                 270
Val Lys Val Asp Tyr Ala Ser Val Pro Asp Ala Leu His Met Met His
        275                 280                 285
Gln Phe Asp Pro Pro Arg Tyr Val Glu Ile Phe Thr Gln Trp Ala Ala
    290                 295                 300
Thr Leu Ala Ala
305

<210> 47
<211> 309
<212> PRT
<213> Artificial Sequence

<220>
<223> Alpha/beta hydrolase domain

<400> 47

Met Val Thr Ser Pro Ala Leu Arg Asp Val His Val Pro His Ala Tyr
1               5                   10                  15
Pro Glu Gln Gln Val Asp Leu Gly Glu Ile Thr Met Asn Tyr Ala Glu
            20                  25                  30
Ala Gly Asp Pro Gly Arg Pro Ala Val Leu Leu Ile Pro Glu Gln Thr
        35                  40                  45
Gly Ser Trp Trp Ser Tyr Glu Glu Ala Met Gly Leu Leu Ala Glu His
    50                  55                  60
Phe His Val Tyr Ala Val Asp Leu Arg Gly Gln Gly Arg Ser Ser Trp
65                  70                  75                  80
Thr Pro Lys Arg Tyr Ser Leu Asp Asn Phe Gly Asn Asp Leu Val Arg
                85                  90                  95
Phe Ile Ala Leu Val Val Arg Arg Pro Val Val Val Ala Gly Asn Ser
            100                 105                 110
Ser Gly Gly Val Leu Ala Ala Trp Leu Ser Ala Tyr Ser Met Pro Gly
        115                 120                 125
Gln Ile Arg Gly Val Leu Cys Glu Asp Pro Pro Phe Phe Ala Ser Glu
    130                 135                 140
Leu Val Pro Ala His Gly His Ser Val Arg Gln Gly Ala Gly Pro Val
145                 150                 155                 160
Phe Glu Leu Phe Arg Thr Tyr Leu Gly Asp Gln Trp Ser Val Gly Asp
                165                 170                 175
Trp Glu Gly Phe Arg Ser Ala Ala Asp Ala Ser Ala Ser Pro Met Ala
            180                 185                 190
Arg Ser Phe Val Ala Asp Thr Ile Pro Gln His Leu Lys Glu Tyr Asp
        195                 200                 205
Pro Glu Trp Ala Arg Ala Phe Tyr Glu Gly Thr Val Gly Leu Asn Cys
    210                 215                 220
Pro His Glu Arg Met Leu Asn Arg Val Asn Thr Pro Val Leu Leu Thr
225                 230                 235                 240
His His Met Arg Gly Thr Asp Pro Glu Thr Gly Asn Leu Leu Gly Ala
                245                 250                 255
Leu Ser Asp Glu Gln Ala Ala Gln Val Arg Arg Leu Met Glu Ser Ala
            260                 265                 270
Gly Val Lys Val Asp Tyr Glu Ser Val Pro Asp Ala Ser His Met Met
        275                 280                 285
His Gln Ser Asp Pro Ala Arg Tyr Ala Glu Ile Leu Thr Pro Trp Thr
    290                 295                 300
Ala Ala Leu Ala Pro
305

<210> 48
<211> 309
<212> PRT
<213> Artificial Sequence

<220>
<223> Alpha/beta hydrolase domain

<400> 48
Met Val Thr Ser Pro Ala Leu Arg Asp Val His Val Pro His Ala Tyr
1               5                   10                  15
Pro Glu Gln Gln Val Asp Leu Gly Glu Ile Thr Met Asn Tyr Ala Glu
            20                  25                  30
Ala Gly Asp Pro Asp Arg Pro Ala Val Leu Leu Ile Pro Glu Gln Thr
        35                  40                  45
Gly Ser Trp Trp Ser Tyr Glu Glu Ala Met Gly Leu Leu Ala Glu His
    50                  55                  60
Phe His Val Tyr Ala Val Asp Leu Arg Gly Gln Gly Arg Ser Ser Trp
65                  70                  75                  80
Thr Pro Lys Arg Tyr Ser Leu Asp Asn Phe Gly Asn Asp Leu Val Arg
                85                  90                  95

Phe Ile Ala Leu Val Val Lys Arg Pro Val Val Val Ala Gly Asn Ser
            100                 105                 110
Ser Gly Gly Val Leu Ala Ala Trp Leu Ser Ala Tyr Ser Met Pro Gly
        115                 120                 125
Gln Leu Arg Gly Val Leu Cys Glu Asp Pro Pro Phe Phe Ala Ser Glu
    130                 135                 140
Leu Val Pro Ala His Gly His Ser Val Arg Gln Gly Ala Gly Pro Val
145                 150                 155                 160
Phe Glu Leu Phe Arg Thr Tyr Leu Gly Asp Gln Trp Ser Val Ser Asp
                165                 170                 175
Trp Glu Gly Phe Cys Arg Ala Ala Gly Ala Ser Ala Ser Pro Met Ala
            180                 185                 190
Arg Ser Phe Val Ala Asp Gly Ile Pro Gln His Leu Lys Glu Tyr Asp
        195                 200                 205
Pro Glu Trp Ala Arg Ala Phe His Glu Gly Thr Val Gly Leu Asn Cys
    210                 215                 220
Pro His Glu Arg Met Leu Gly Arg Val Asn Thr Pro Val Leu Leu Thr
225                 230                 235                 240
His His Met Arg Gly Thr Asp Pro Glu Thr Gly Asn Leu Leu Gly Ala
                245                 250                 255
Leu Ser Asp Glu Gln Ala Ala Gln Ala Arg Leu Leu Met Glu Ser Ala
            260                 265                 270
Gly Val Arg Val Asp Tyr Glu Ser Val Pro Asp Ala Ser His Met Met
        275                 280                 285
His Gln Ser Asp Pro Ala Arg Tyr Ala Glu Ile Phe Thr Arg Trp Ala
    290                 295                 300
Ala Ala Leu Ala Pro
305

<210> 49
<211> 309
<212> PRT
<213> Artificial Sequence

<220>
<223> Alpha/beta hydrolase domain

<400> 49
Met Val Thr Ser Pro Ala Leu Arg Asp Val His Val Pro His Ala Tyr
1               5                   10                  15
Pro Glu Gln Gln Val Asp Leu Gly Glu Ile Thr Met Asn Tyr Ala Glu
            20                  25                  30
Ala Gly Asp Pro Gly Arg Pro Ala Val Leu Leu Ile Pro Glu Gln Thr
        35                  40                  45
Gly Ser Trp Trp Ser Tyr Glu Glu Ala Met Gly Leu Leu Ala Glu His
    50                  55                  60
Phe His Val Tyr Ala Val Asp Leu Arg Gly Gln Gly Arg Ser Ser Trp
65                  70                  75                  80
Thr Pro Lys Arg Tyr Ser Leu Asp Asn Phe Gly Asn Asp Leu Val Arg
                85                  90                  95
Phe Met Ala Leu Val Val Arg Arg Pro Val Val Val Ala Gly Asn Ser
            100                 105                 110
Ser Gly Gly Val Leu Ala Ala Trp Leu Ser Ala Tyr Ser Met Pro Gly
        115                 120                 125
Gln Ile Arg Gly Val Leu Cys Glu Asp Pro Pro Phe Phe Ala Ser Glu
    130                 135                 140
Leu Val Pro Ala His Gly His Ser Val Arg Gln Gly Ala Gly Pro Val
145                 150                 155                 160
Phe Glu Leu Phe Arg Thr Tyr Leu Gly Asp Gln Trp Ser Val Gly Asp
                165                 170                 175
Trp Glu Gly Phe Arg Ser Ala Ala Gly Ala Ser Ala Ser Pro Met Ala
            180                 185                 190

Arg Ser Phe Val Ala Asp Thr Ile Pro Gln His Leu Lys Glu Tyr Asp
        195                 200                 205
Pro Glu Trp Ala Arg Ala Phe Tyr Glu Gly Thr Val Gly Leu Asn Cys
    210                 215                 220
Pro His Glu Arg Met Leu Asn Arg Val Asn Thr Pro Val Leu Leu Thr
225                 230                 235                 240
His His Met Arg Gly Thr Asp Pro Glu Thr Gly Asn Leu Leu Gly Ala
                245                 250                 255
Leu Ser Asp Glu Gln Ala Ala Gln Ala Arg Arg Leu Met Glu Ser Ala
            260                 265                 270
Gly Val Lys Val Asp Tyr Glu Ser Val Pro Asp Ala Ser His Met Met
        275                 280                 285
His Gln Ser Asp Pro Ala Arg Tyr Ala Glu Ile Leu Thr Pro Trp Ala
    290                 295                 300
Ala Ala Leu Ala Pro
305

<210> 50
<211> 328
<212> PRT
<213> Artificial Sequence

<220>
<223> Alpha/beta hydrolase domain

<400> 50
Met Ala Glu Glu Gly Thr Arg Ser Glu Ala Ala Asp Ala Ala Thr Gln
1               5                   10                  15
Ala Arg Gln Leu Pro Asp Ser Arg Asn Ile Phe Val Ser His Arg Phe
            20                  25                  30
Pro Glu Arg Gln Val Asp Leu Gly Glu Val Val Met Asn Phe Ala Glu
        35                  40                  45
Ala Gly Ser Pro Asp Asn Pro Ala Leu Leu Leu Leu Pro Glu Gln Thr
    50                  55                  60
Gly Ser Trp Trp Ser Tyr Glu Pro Val Met Gly Leu Leu Ala Glu Asn
65                  70                  75                  80
Phe His Val Phe Ala Val Asp Ile Arg Gly Gln Gly Arg Ser Thr Trp
                85                  90                  95
Thr Pro Arg Arg Tyr Ser Leu Asp Asn Phe Gly Asn Asp Leu Val Arg
            100                 105                 110
Phe Ile Ala Leu Val Ile Lys Arg Pro Val Val Val Ala Gly Asn Ser
        115                 120                 125
Ser Gly Gly Leu Leu Ala Ala Trp Leu Ser Ala Tyr Ala Met Pro Gly
    130                 135                 140
Gln Ile Arg Ala Ala Leu Cys Glu Asp Ala Pro Phe Phe Ala Ser Glu
145                 150                 155                 160
Leu Val Pro Ala Tyr Gly His Ser Val Leu Gln Ala Ala Gly Pro Ala
                165                 170                 175
Phe Glu Leu Tyr Arg Asp Phe Leu Gly Asp Gln Trp Ser Ile Gly Asp
            180                 185                 190
Trp Lys Gly Phe Val Glu Ala Ala Lys Ala Ser Pro Ala Lys Ala Met
        195                 200                 205
Gln Leu Phe Pro Thr Pro Asp Glu Ala Pro Gln Asn Leu Lys Glu Tyr
    210                 215                 220
Asp Pro Glu Trp Gly Arg Ala Phe Phe Glu Gly Thr Val Ala Leu His
225                 230                 235                 240
Cys Pro His Asp Arg Met Leu Ser Gln Val Lys Thr Pro Ile Leu Ile
                245                 250                 255
Thr His His Ala Arg Thr Ile Asp Pro Glu Thr Gly Glu Leu Leu Gly
            260                 265                 270
Ala Leu Ser Asp Leu Gln Ala Glu His Ala Gln Asp Ile Ile Arg Ser
        275                 280                 285

Ala Gly Val Arg Val Asp Tyr Gln Ser His Pro Asp Ala Leu His Met
    290                 295                 300
Met His Leu Phe Asp Pro Ala Arg Tyr Ala Glu Ile Leu Thr Ser Trp
305                 310                 315                 320
Ser Ala Thr Leu Pro Ala Asn Asp
                325